# EUROPEAN PATENT APPLICATION

(11) **EP 4 410 352 A1**
(43) Date of publication of application: **07.08.2024**
(21) Application number: 22872553.7
(22) Date of filing: 28.07.2022
(51) Int. Cl.: A61M 25/082, A61B 34/30

(54) **MEDICAL DEVICE**

(30) Priority: 27.09.2021 JP 2021156229
(71) Applicant: CANON KABUSHIKI KAISHA, Tokyo 146-8501 (JP)
(72) Inventor: NIIKAWA, Yusuke, Tokyo 146-8501 (JP); IWASAWA, Ryo, Tokyo 146-8501 (JP); NOGUCHI, Tomio, Tokyo 146-8501 (JP)
(74) Representative: TBK
(86) International application number: PCT/JP2022/029160
(87) International publication number: WO 2023/047797

(57) **Abstract**

The medical apparatus (1) includes a driving source (M), a base unit (200) including a coupling portion (21c) connected to the driving source (M), a bendable unit (100) including a bending portion (12), and a linear member (W) configured to bend the bending portion (12) by being driven by the driving source (M) through the coupling portion (21c), an operation portion (400), and a deceleration mechanism (350) including an output member (252) that is driven by a force transmitted from the operation portion (400). The coupling portion (21c) includes a retaining portion (21ch) configured to be transited between a retaining state and a releasing state, and a switching portion (21cp) configured to be moved by the output member (252) and configured to switch the retaining portion (21ch) between the retaining state and the releasing state. In a case where the operation portion (400) is moved for a first movement amount, the output member (252) is configured to be moved for a second movement amount that is smaller than the first movement amount.

## Description

### [Technical Field]

The present invention relates to a medical apparatus having a bending portion which is bendable.

### [Background Art]

Patent Literature 1 discloses a medical device that includes an operated portion having a deformation portion, and an operation portion for deforming the deformation portion, wherein the operated portion and the operation portion are detachably attached. Wires for deforming the deformation portion are disposed on the operated portion, and an operated portion-side magnet is fixed to the wires. Wires wound around a pulley are disposed at the operation portion, and an operation portion-side magnet is fixed to the wires. In a state where the operated portion is connected to the operation portion, the operated portion-side magnet and the operation portion-side magnet are drawn to each other, and the wires disposed on the operation portion are interlocked with the wires disposed on the operated portion.

### [Cited Reference]

### [Patent Literature]

[PTL1] Japanese Patent Application Laid-Open Publication No. 2013-248116

### [Summary of Invention]

### [Technical Problem]

According to the configuration disclosed in Patent Literature 1, however, when detaching the operated portion from the operation portion, the user must pull the operated portion against an attractive force between the operated portion-side magnet and the operation portion-side magnet attracted to each other.

### [Solution to Problem]

According to one aspect of the present invention, a medical apparatus includes a driving source, a base unit including a coupling portion connected to the driving source, a bendable unit removably attached to the base unit, the bendable unit including a bending portion which is bendable, and a linear member configured to be coupled to the coupling portion and configured to bend the bending portion by being driven by the driving source through the coupling portion, an operation portion, and a deceleration mechanism including an output member that is driven by a force transmitted from the operation portion. The coupling portion includes a retaining portion configured to be transited between a retaining state in which the retaining portion retains the linear member in a state where the bendable unit is attached to the base unit and a releasing state in which retaining of the linear member is released and a switching portion configured to be moved by the output member and configured to switch the retaining portion between the retaining state and the releasing state. In a case where the operation portion is moved for a first movement amount, the output member is configured to be moved for a second movement amount that is smaller than the first movement amount.

### [Advantageous Effects of Invention]

According to the present invention, the operation force of the operation portion may be reduced.

Other features and advantages of the present invention will become apparent from the following descriptions with reference to the accompanying drawings. In the accompanying drawings, the same or similar configurations are denoted with the same reference numbers.

### [Brief Description of Drawings]

[FIG. 1] FIG. 1 is a general view of a medical system.
[FIG. 2] FIG. 2 is a perspective view illustrating a medical apparatus and a supporting base.
[FIG. 3A] FIG. 3A is a general perspective view illustrating a catheter.
[FIG. 3B] FIG. 3B is an enlarged perspective view illustrating the catheter.
[FIG. 4A] FIG. 4A is a perspective view illustrating a catheter unit in a state where a wire cover is at a cover position.
[FIG. 4B] FIG. 4B is a perspective view illustrating a catheter unit in a state where the wire cover is at an exposure position.
[FIG. 5A] FIG. 5A is a perspective view illustrating an internal configuration of a base unit.
[FIG. 5B] FIG. 5B is a side view illustrating the internal configuration of the base unit.
[FIG. 5C] FIG. 5C is a view illustrating the base unit along an attaching and detaching direction.
[FIG. 6A] FIG. 6A is a perspective view illustrating a driving source, a coupling portion, and a driving wire.
[FIG. 6B] FIG. 6B is an enlarged view illustrating the coupling portion and the driving wire.
[FIG. 6C] FIG. 6C is a perspective view illustrating a wire driving portion, a coupling device, and a bending driving portion.
[FIG. 7A] FIG. 7A is a cross-sectional view illustrating a state before the catheter unit is attached to a base unit.
[FIG. 7B] FIG. 7B is a cross-sectional view illustrating a state after the catheter unit has been attached to the base unit.
[FIG. 8] FIG. 8 is a perspective view illustrating a connecting part of the base unit to the catheter unit.
[FIG. 9] FIG. 9 is a perspective view illustrating a planetary gear mechanism disposed in the base unit.
[FIG. 10] FIG. 10 is an exploded perspective view illustrating an operation portion and the planetary gear mechanism.
[FIG. 11] FIG. 11 is an exploded perspective view illustrating the planetary gear mechanism.
[FIG. 12] FIG. 12 is a cross-sectional view of the operation portion and the base unit cut along a rotation shaft of the operation portion.
[FIG. 13] FIG. 13 is a cross-sectional view illustrating a 13A-13A cross-section of FIG. 12.
[FIG. 14A] FIG. 14A is a cross-sectional view of the catheter unit and the base unit cut along the rotation shaft of the operation portion.
[FIG. 14B] FIG. 14B is a cross-sectional view of the base unit cut along a direction orthogonal to the rotation shaft.
[FIG. 15] FIG. 15 is a view illustrating a fixing of a driving wire by a coupling portion.
[FIG. 16] FIG. 16 is a view illustrating the fixing of the driving wire by the coupling portion.
[FIG. 17] FIG. 17 is a view illustrating the fixing of the driving wire by the coupling portion.
[FIG. 18] FIG. 18 is a view illustrating the fixing of the driving wire by the coupling portion.
[FIG. 19] FIG. 19 is a view illustrating the fixing of the driving wire by the coupling portion.
[FIG. 20A] FIG. 20A is a cross-sectional view of the catheter unit.
[FIG. 20B] FIG. 20B is a perspective view of a button.
[FIG. 20C] FIG. 20C is a perspective view of the base unit.
[FIG. 21A] FIG. 21A is a view illustrating a state where the operation portion is at a removal position.
[FIG. 21B] FIG. 21B is a view illustrating a state where the operation portion is at a release position.
[FIG. 21C] FIG. 21C is a view illustrating a state where the operation portion is at a fixed position.
[FIG. 22A] FIG. 22A is a cross-sectional view illustrating a state where the operation portion is at the removal position.
[FIG. 22B] FIG. 22B is a cross-sectional view illustrating a state where the operation portion is at the release position.
[FIG. 22C] FIG. 22C is a cross-sectional view illustrating a state where the operation portion is at the fixed position.
[FIG. 23A] FIG. 23A is a skeleton drawing illustrating a planet-type planetary gear mechanism according to a first embodiment.
[FIG. 23B] FIG. 23B is a skeleton drawing illustrating a solar-type planetary gear mechanism according to a modified example of the first embodiment.
[FIG. 23C] FIG. 23C is a skeleton drawing illustrating a star-type planetary gear mechanism according to another modified example of the first embodiment.
[FIG. 24] FIG. 24 is a perspective view illustrating a stepped gear mechanism according to a second embodiment.
[FIG. 25] FIG. 25 is an exploded perspective view illustrating the stepped gear mechanism.

### [Description of Embodiments]

Embodiments according to the present disclosure will be described with reference to the drawings.

### [First Embodiment]

### <Medical System and Medical Apparatus>

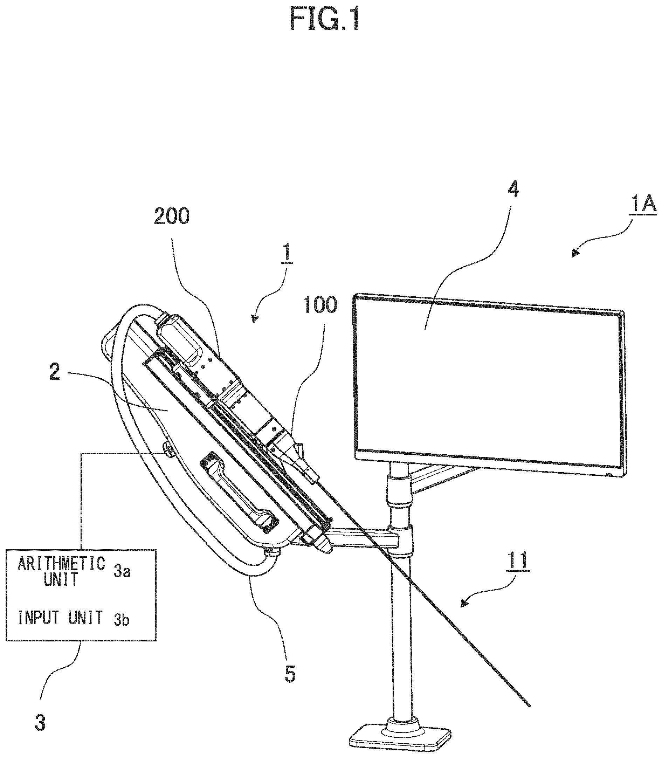

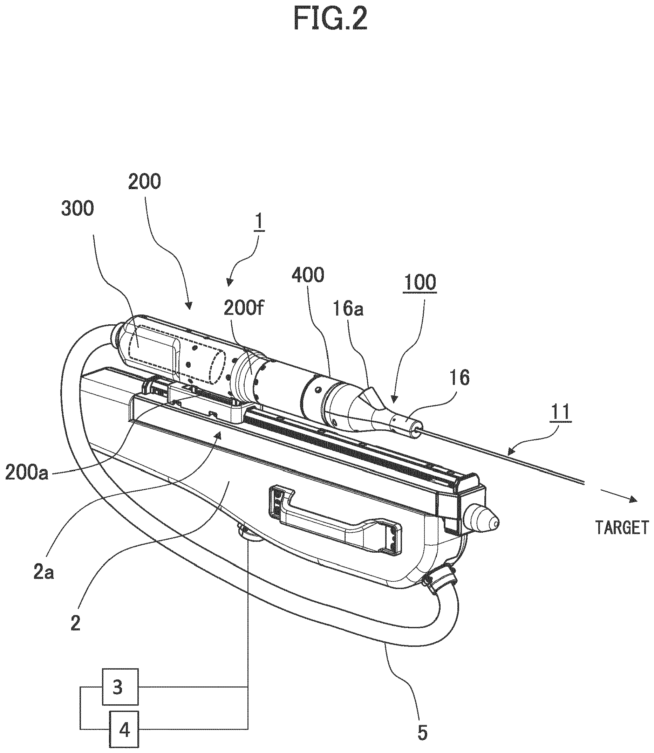

A medical system 1A and a medical apparatus 1 according to a first embodiment will be described with reference to FIGs. 1 and 2. FIG. 1 is a general view of the medical system 1A. FIG. 2 is a perspective view illustrating the medical apparatus 1 and a supporting base 2.

The medical system 1A is equipped with the medical apparatus 1, the supporting base 2 for attaching the medical apparatus 1, and a control portion (control device) 3 for controlling the medical apparatus 1. In the present embodiment, the medical system 1A is equipped with a monitor 4 serving as a display device.

The medical apparatus 1 is equipped with a catheter unit (bendable unit) 100 equipped with a catheter 11 serving as a bendable body, and a base unit (drive unit, attached unit) 200. The catheter unit 100 is configured attachably/detachably with respect to the base unit 200.

In the present embodiment, a user of the medical system 1A and the medical apparatus 1 may insert the catheter 11 to an inner side of a target to perform operations such as observation of the inner side of the target, collecting of various samples from the inner side of the target, and treatment of the inner side of the target. As an embodiment, the user may insert the catheter 11 to the inner side of a patient serving as the target. Specifically, by inserting the catheter through an oral cavity or a nasal cavity of the patient into a bronchus, operations such as observation, collection, and excision of pulmonary tissues may be performed.

The catheter 11 may be used as a guide (sheath) for guiding a medical device for performing the above-mentioned operations. An endoscope, a forceps, and an ablation device are examples of the medical device (tool). Further, the catheter 11 itself may have the functions as the above-described medical devices, and in that case, the catheter 11 is not limited to a tubular shape, and it may be a cylindrical shape, for example.

In the present embodiment, a control portion 3 includes an arithmetic unit 3a, and an input unit 3b. The input unit 3b receives commands and inputs for operating the catheter 11. The arithmetic unit 3a includes a storage for storing programs for controlling the catheter and various data, a random access memory, and a central processing unit for executing the programs. Further, the control portion 3 may be equipped with an output portion for outputting signals for display images on the monitor 4.

As illustrated in FIG. 2, according to the present embodiment, the medical apparatus 1 is connected electrically through a cable 5 coupling the base unit 200 and the supporting base 2 of the medical apparatus 1 and the supporting base 2 to the control portion 3. Further, the medical apparatus 1 and the control portion 3 may be connected directly by a cable. The medical apparatus 1 and the control portion 3 may be connected wirelessly.

The medical apparatus 1 is detachably attached through the base unit 200 to the supporting base 2. More specifically, the medical apparatus 1 has an attachment portion (connection portion) 200a of the base unit 200 removably attached to a moving stage (receiving portion) 2a of the supporting base 2. Even in a state where the attachment portion 200a of the medical apparatus 1 is removed from the moving stage 2a, the connection between the medical apparatus 1 and the control portion 3 is maintained such that the medical apparatus 1 may be controlled by the control portion 3. In the present embodiment, even in a state where the attachment portion 200a of the medical apparatus 1 is removed from the moving stage 2a, the medical apparatus 1 and the supporting base 2 are connected by the cable 5.

The user may move the medical apparatus 1 manually in a state where the medical apparatus 1 is removed from the supporting base 2 (in a state where the medical apparatus 1 is removed from the moving stage 2a), and may insert the catheter 11 to the inner side of the target.

The user may use the medical apparatus 1 in a state where the catheter 11 is inserted to the target and the medical apparatus 1 is attached to the supporting base 2. Specifically, in a state where the medical apparatus 1 is attached to the moving stage 2a, by moving the moving stage 2a, the medical apparatus 1 is moved. Then, an action of moving the catheter 11 toward a direction inserting the same to the target, and an action of moving the catheter 11 toward a direction drawing the same out of the target are performed. The movement of the moving stage 2a is controlled by the control portion 3.

The attachment portion 200a of the base unit 200 is equipped with a release switch and a removal switch not shown. In a state where the attachment portion 200a is attached to the moving stage 2a, the user may move the medical apparatus 1 manually along the guiding direction of the moving stage 2a while pressing the release switch continuously. That is, the moving stage 2a is equipped with a guiding configuration for guiding the movement of the medical apparatus 1. When the user stops pressing the release switch, the medical apparatus 1 is fixed to the moving stage 2a. Meanwhile, when the removal switch is pressed in a state where the attachment portion 200a is attached to the moving stage 2a, the user may remove the medical apparatus 1 from the moving stage 2a.

Incidentally, one switch may have the function of a release switch and the function of a removal switch. Further, if the release switch is provided with a function to switch a pressed state and a non-pressed state of the release switch, the user will not be required to keep pressing the release switch when manually sliding and moving the medical apparatus 1.

In a state where the attachment portion 200a is attached to the moving stage 2a and the release switch and the removal switch are not pressed, the medical apparatus 1 is fixed to the moving stage 2a, and moved by the moving stage 2a that is driven by a motor not shown.

The medical apparatus 1 is equipped with a wire driving portion (linear member driving portion, line driving portion, body driving portion) 300 for driving the catheter 11. In the present embodiment, the medical apparatus 1 is a robot catheter apparatus that drives the catheter 11 by the wire driving portion 300 controlled by the control portion 3.

The control portion 3 may control the wire driving portion 300 and perform an action to bend the catheter 11. In the present embodiment, the wire driving portion 300 is disposed in the base unit 200. More specifically, the base unit 200 is equipped with a base casing 200f for storing the wire driving portion 300. That is, the base unit 200 is provided with the wire driving portion 300. The wire driving portion 300 and the base unit 200 may collectively be referred to as a catheter driving apparatus (base device, main body).

Regarding the extending direction of the catheter 11, the end portion at which a tip of the catheter 11 inserted to the target is arranged is called a distal end. An opposite side of the distal end in the extending direction of the catheter 11 is called a proximal end.

The catheter unit 100 includes a proximal end cover 16 for covering a proximal end side of the catheter 11. The proximal end cover 16 includes a tool hole 16a. The catheter 11 may insert the medical device through the tool hole 16a.

As described above, in the present embodiment, the catheter 11 has a function as a guide device for guiding the medical device to a desired position on the inner side of the target.

For example, in a state where the endoscope is inserted to the catheter 11, the catheter 11 is inserted to a target position on the inner side of the target. In this state, at least one of the manual operation of the user, the movement of the moving stage 2a, and the driving of the catheter 11 by the wire driving portion 300 is used. After the catheter 11 reaches the target position, the endoscope is drawn out of the catheter 11 through the tool hole 16a. Then, the medical device is inserted through the tool hole 16a, and operations such as collecting of various samples from the inner side of the target and treatment of the inner side of the target are performed.

As described later, the catheter unit 100 is attached removably to the catheter driving apparatus (base device, body), more specifically, to the base unit 200. After using the medical apparatus 1, the user removes the catheter unit 100 from the base unit 200 and attaches a new catheter unit 100 to the base unit 200, before using the medical apparatus 1 again. That is, the catheter unit 100 may be used as a disposable unit. The term disposable means that the catheter unit 100 used for one treatment is disposed after use. Thereby, it becomes possible to prevent reuse of the catheter unit 100 and to thereby constantly maintain the medical apparatus 1 in a clean state.

As illustrated in FIG. 2, the medical apparatus 1 includes an operation portion 400. In the present embodiment, the operation portion 400 is provided in the catheter unit 100. The operation portion 400 is operated by the user when performing fixing of the catheter unit 100 to the base unit 200 and removing the catheter unit 100 from the base unit 200.

By connecting the endoscope inserted to the catheter 11 and the monitor 4, an image taken by the endoscope may be displayed on the monitor 4. Further, by connecting the monitor 4 and the control portion 3, the information related to the state of the medical apparatus 1 and the control of the medical apparatus 1 may be displayed on the monitor 4. For example, information related to the position of the catheter 11 in the inner side of the target or the navigation of the catheter 11 on the inner side of the target may be displayed on the monitor 4. The monitor 4, the control portion 3, and the endoscope may be connected in a wired manner or connected wirelessly. Further, the monitor 4 and the control portion 3 may be connected via the supporting base 2.

### <Catheter>

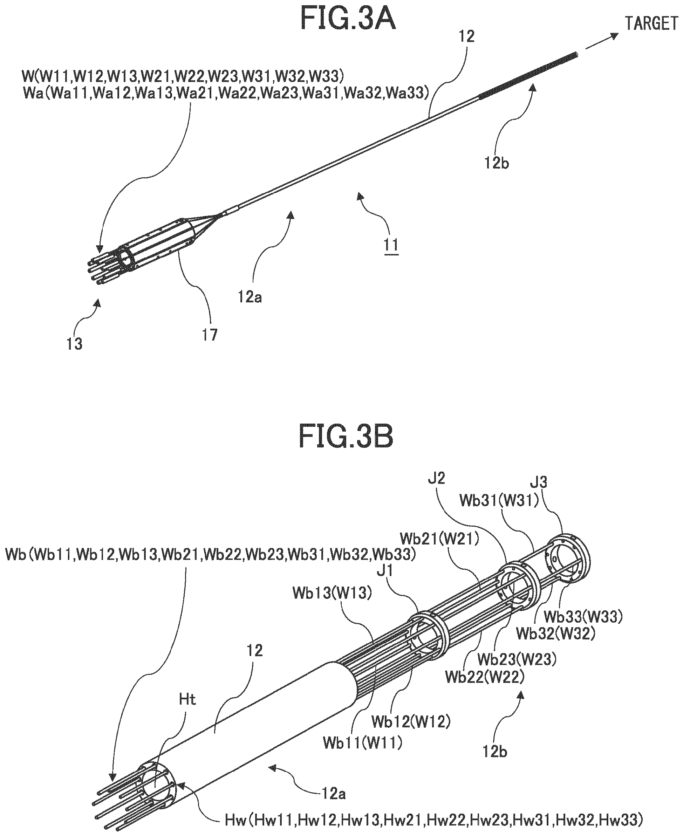

The catheter 11 serving as a bendable body will be described with reference to FIGs. 3A and 3B. FIGs. 3A and 3B are each an explanatory view of the catheter 11. FIG. 3A is an explanatory view of the entire catheter 11. FIG. 3B is an enlarged view of the catheter 11.

The catheter 11 is equipped with a bending portion (bending body, catheter body) 12, and a bending driving portion (catheter driving portion) 13 configured to bend the bending portion 12. The bending driving portion 13 is configured to bend the bending portion 12 by receiving a driving force of the wire driving portion 300 through a coupling device 21 described later.

The catheter 11 is extended along the inserting direction of the catheter 11 to the target. The extending direction (longitudinal direction) of the catheter 11 is the same as the extending direction (longitudinal direction) of the bending portion 12, and the extending direction (longitudinal direction) of first to ninth driving wires (W 11 to W33) described later.

The bending driving portion 13 includes a plurality of driving wires (driving lines, linear members, linear actuators) connected to the bending portion 12. Specifically, the bending driving portion 13 includes a first driving wire W11, a second driving wire W12, a third driving wire W13, a fourth driving wire W21, a fifth driving wire W22, a sixth driving wire W23, a seventh driving wire W31, an eighth driving wire W32, and a ninth driving wire W33.

Each of the first to ninth driving wires (W11 to W33) includes a retained portion (retained shaft, rod) Wa. Specifically, the first driving wire W11 includes a first retained portion Wa11. The second driving wire W12 includes a second retained portion Wa12. The third driving wire W13 includes a third retained portion Wa13. The fourth driving wire W21 includes a fourth retained portion Wa21. The fifth driving wire W22 includes a fifth retained portion Wa22. The sixth driving wire W23 includes a sixth retained portion Wa23. The seventh driving wire W31 includes a seventh retained portion Wa31. The eighth driving wire W32 includes an eighth retained portion Wa32. The ninth driving wire W33 includes a ninth retained portion Wa33.

In the present embodiment, each of the first to ninth retained portions (Wa11 to Wa33) has the same shape.

Each of the first to ninth driving wires (W11 to W33) has a wire body (wire member, line body, linear body) Wb having flexibility. The wire body Wb described here is a member capable of allowing an object connected therethrough to be pushed and pulled, and it has a certain level of stiffness. Meanwhile, it is a member that may be deformed from a linear shape so as to allow the bending portion 12 to bend. The first driving wire W11 includes a first wire body Wb11. The second driving wire W12 includes a second wire body Wb12. The third driving wire W13 includes a third wire body Wb13. The fourth driving wire W21 includes a fourth wire body Wb21. The fifth driving wire W22 includes a fifth wire body Wb22. The sixth driving wire W23 includes a sixth wire body Wb23. The seventh driving wire W31 includes a seventh wire body Wb31. The eighth driving wire W32 includes an eighth wire body Wb32. The ninth driving wire W33 includes a ninth wire body Wb33.

In the present embodiment, the first to third wire bodies (Wb11 to Wb13) each have the same shape. The fourth to sixth wire bodies (Wb21 to Wb23) each have the same shape. The seventh to ninth wire bodies (Wb31 to Wb33) each have the same shape. In the present embodiment, the first to ninth wire bodies (Wb11 to Wb33) have the same shape, except for their lengths.

The first to ninth retained portions (Wa11 to Wa33) are attached to proximal ends of the first to ninth wire bodies (Wb11 to Wb33). The first to ninth driving wires (W11 to W33) are inserted and fixed to the bending portion 12 through a wire guide 17.

In the present embodiment, the material of each of the first to ninth wire bodies (Wb11 to Wb33) is metal. However, the material of each of the first to ninth wire bodies (Wb11 to Wb33) may be resin. Further, the material of each of the first to ninth wire bodies (Wb11 to Wb33) may include metal and resin.

An arbitrary one among the first to ninth driving wires (W11 to W33) may be called a driving wire W serving as a linear member. In the present embodiment, the first to ninth driving wires (W11 to W33) each have the same shape, except for the lengths of the first to ninth wire bodies (Wb11 to Wb33).

In the present embodiment, the bending portion 12 is a tubular member having flexibility and equipped with a passage Ht through which the medical device may be inserted.

A plurality of wire holes for passing each of the first to ninth driving wires (W11 to W33) are provided on a wall surface of the bending portion 12. Specifically, a first wire hole Hw11, a second wire hole Hw12, and a third wire hole Hw13 are provided on the wall surface of the bending portion 12. Further, a fourth wire hole Hw21, a fifth wire hole Hw22, and a sixth wire hole Hw23 are provided on the wall surface of the bending portion 12. Furter, a seventh wire hole Hw31, an eighth wire hole Hw32, and a ninth wire hole Hw33 are provided on the wall surface of the bending portion 12. Each of the first to ninth wire holes Hw (Hw11 to Hw33) corresponds to each of the first to ninth driving wires (W11 to W33). The numeral after the reference Hw indicates the number of the corresponding driving wire. For example, the first driving wire W11 is inserted to the first wire hole Hw11.

Among the first to ninth wire holes (Hw11 to Hw33), an arbitrary one may be called a wire hole Hw. In the present embodiment, the first to ninth wire holes (Hw11 to Hw33) each have the same shape.

The bending portion 12 includes an intermediate region 12a and a bending region 12b. The bending region 12b is arranged at a distal end of the bending portion 12, and on the bending region 12b are disposed a first guide ring J1, a second guide ring J2, and a third guide ring J3. The bending region 12b refers to a region where a magnitude and a direction of the bend of the being portion 12 may be controlled by moving the first guide ring J1, the second guide ring J2, and the third guide ring J3 through the bending driving portion 13. FIG. 3B is a view in which a part of the bending portion 12 covering the first to third guide rings (J1 to J3) is not shown.

In the present embodiment, the bending portion 12 is equipped with a plurality of auxiliary rings (not shown). In the bending region 12b, the first guide ring J1, the second guide ring J2, and the third guide ring J3 are fixed to a wall surface of the bending portion 12. In the present embodiment, the plurality of auxiliary rings are arranged between the first guide ring J1 and the second guide ring J2, and between the second guide ring J2 and the third guide ring J3.

The medical device is guided by the passage Ht, the first to third guide rings (J1 to J3), and the plurality of auxiliary rings to the tip of the catheter 11.

Each of the first to ninth driving wires (W11 to W33) are fixed to each of the first to third guide rings (J1 to J3) through the intermediate region 12a. Specifically, the first driving wire W11, the second driving wire W12, and the third driving wire W13 are fixed to the first guide ring J1. The fourth driving wire W21, the fifth driving wire W22, and the sixth driving wire W23 are passed through the first guide ring J1 and the plurality of auxiliary rings and fixed to the second guide ring J2. The seventh driving wire W31, the eighth driving wire W32, and the ninth driving wire W33 are passed through the first guide ring J1, the second guide ring J2, and the plurality of auxiliary rings and fixed to the third guide ring J3.

The medical apparatus 1 may bend the bending portion 12 toward a direction intersecting the extending direction of the catheter 11 by driving the bending driving portion 13 through the wire driving portion 300. Specifically, the bending region 12b of the bending portion 12 may be bent in the direction intersecting the extending direction through the first to third guide rings (J1 to J3) by moving each of the first to ninth driving wires (W11 to W33) in the extending direction of the bending portion 12.

The user may use at least one of the movement of the medical apparatus 1 either manually or through the moving stage 2a and the bending of the bending portion 12 to insert the catheter 11 to a target portion on the inner side of the target.

In the present embodiment, the first to third guide rings (J1 to J3) are moved by the first to ninth driving wires (W11 to W33) to bend the bending portion 12, but the present technique is not limited to this configuration. Any one or two of the first to third guide rings (J1 to J3) and the driving wire fixed thereto may be omitted.

For example, a configuration may be adopted in which the catheter 11 includes the seventh to ninth driving wires (W31 to W33) and the third guide ring J3, and wherein the first to sixth driving wires (W11 to W23) and the first and second guide rings (J1 to J2) are omitted. Further, a configuration may be adopted in which the catheter 11 includes the fourth to ninth driving wires (W21 to W33) and the second to third guide rings (J2 to J3), and wherein the first to third driving wires (W11 to W13) and the first guide ring J1 are omitted.

Further, a configuration may be adopted in which the catheter 11 drives one guide ring with two driving wires. Similarly, according to this case, the number of the guide rings may be one, or more than one.

### <Catheter Unit>

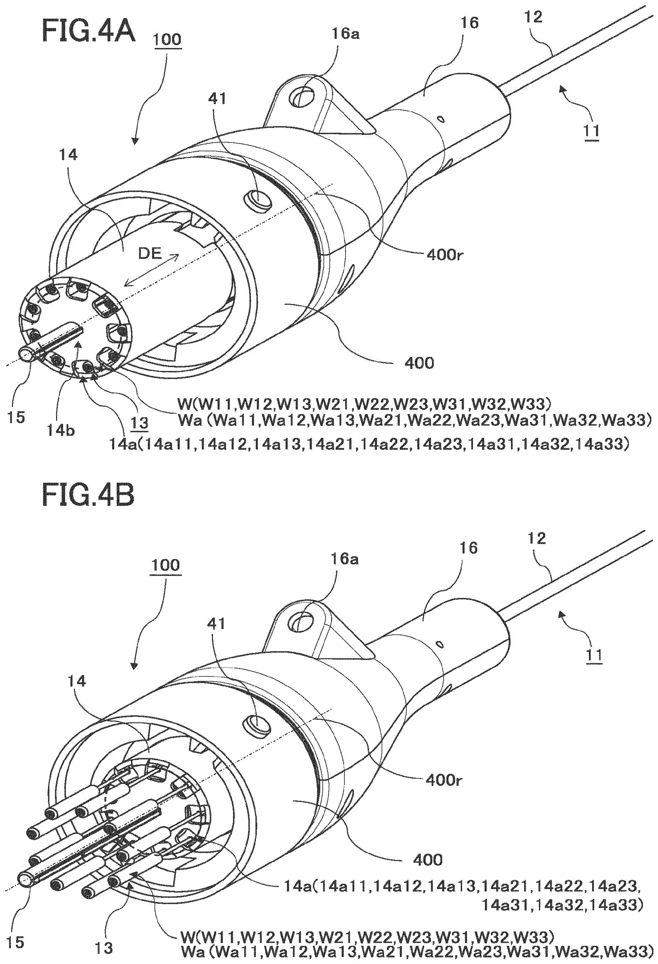

The catheter unit 100 will be described with reference to FIGs. 4A and 4B. FIGs. 4A and 4B are explanatory views of the catheter unit 100. FIG. 4A is an explanatory view of the catheter unit 100 in a state where a wire cover 14 described later is in a cover position. FIG. 4B is an explanatory view of the catheter unit 100 in a state where the wire cover 14 described later is in an exposure position.

The catheter unit 100 includes the catheter 11 including the bending portion 12 and the bending driving portion 13, and the proximal end cover 16 supporting the proximal end of the catheter 11. The catheter unit 100 is equipped with a cover (wire cover) 14 for covering and protecting the first to ninth driving wires (W11 to W33) serving as the plurality of driving wires.

The catheter unit 100 may be attached to and detached from the base unit 200 along an attaching and detaching direction DE. The attaching direction of the catheter unit 100 to the base unit 200 and the removal direction of the catheter unit 100 from the base unit 200 are parallel with the attaching and detaching direction DE.

The proximal end cover (frame body, bending portion casing, catheter casing) 16 is a cover that covers a part of the catheter 11. The proximal end cover 16 includes the tool hole 16a for inserting the medical device to the passage Ht of the bending portion 12.

A plurality of exposing holes (wire cover holes, cover holes) for passing through each of the first to ninth driving wires (W11 to W33) are provided on the wire cover 14. The wire cover 14 is provided with a first exposing hole 14a11, a second exposing hole 14a12, a third exposing hole 14a13, a fourth exposing hole 14a21, a fifth exposing hole 14a22, a sixth exposing hole 14a23, a seventh exposing hole 14a31, an eighth exposing hole 14a32, and a ninth exposing hole 14a33. Each of the first to ninth exposing holes (14a11 to 14a33) corresponds to each of the first to ninth driving wires (W11 to W33). The numeral after the reference 14a indicates the number of the corresponding driving wire. For example, the first driving wire W11 is inserted to the first exposing hole 14a11.

An arbitrary one among the first to ninth exposing holes (14a11 to 14a33) may be called the exposing hole 14a. In the present embodiment, the first to ninth exposing holes (14a11 to 14a33) each have the same shape.

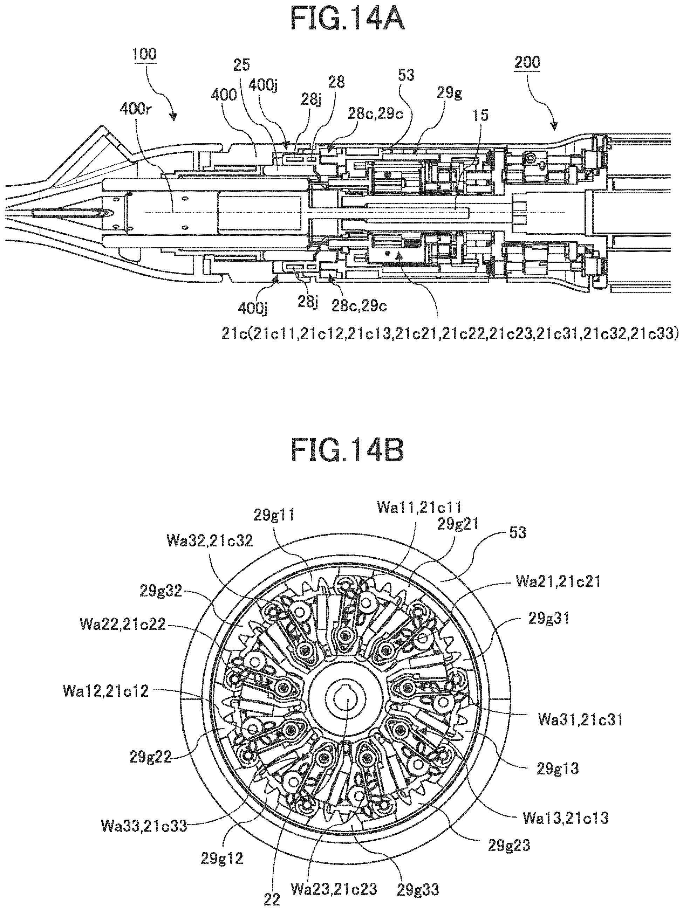

The wire cover 14 may be moved between a cover position (refer to FIG. 14A) covering the first to ninth driving wires (W11 to W33) and a cover retracting position (refer to FIG. 14B) retracted from the cover position. The cover retracting position may also be called an exposure position exposing the first to ninth driving wires (W11 to W33).

Before the catheter unit 100 is attached to the base unit 200, the wire cover 14 is positioned at the cover position. When the catheter unit 100 is attached to the base unit 200, the wire cover 14 is moved from the cover position to the exposure position along the attaching and detaching direction DE.

In the present embodiment, after the wire cover 14 is moved from the cover position to the exposure position, the wire cover 14 is held at the exposure position. Therefore, after attaching the catheter unit 100 to the base unit 200, the wire cover 14 is held at the exposure position even when the catheter unit 100 is removed from the base unit 200.

However, the wire cover 14 may be configured to be returned to the cover position after being moved from the cover position to the exposure position. For example, the catheter unit 100 may be provided with an urging member for urging the wire cover 14 from the exposure position to the cover position. In this case, after attaching the catheter unit 100 to the base unit 200, when the catheter unit 100 is removed from the base unit 200, the wire cover 14 is moved from the exposure position to the cover position.

When the wire cover 14 is at the exposure position, the first to ninth retained portions (Wa11 to Wa33) of the first to ninth driving wires (W11 to W33) are exposed. As a result, the coupling between the bending driving portion 13 and the coupling device 21 described later is permitted. When the wire cover 14 is positioned at the exposure position, the first to ninth retained portions (Wa11 to Wa33) of the first to ninth driving wires (W11 to W33) are projected through the first to ninth exposing holes (14a11 to 14a33). More specifically, the first to ninth retained portions (Wa11 to Wa33) are projected through the first to ninth exposing holes (14a11 to 14a33) toward an attaching direction Da described later.

As illustrated in FIG. 4B, each of the first to ninth driving wires (W11 to W33) are arranged along a circle (virtual circle) having a predetermined radius.

In the present embodiment, the catheter unit 100 includes a key shaft (key, catheter-side key) 15. In the present embodiment, the key shaft 15 extends toward the attaching and detaching direction DE. The wire cover 14 is provided with a shaft hole 14b through which the key shaft 15 passes. The key shaft 15 is engageable with a key receiving portion 22 described later. By the key shaft 15 engaging with the key receiving portion 22, the movement of the catheter unit 100 with respect to the base unit 200 is limited within a predetermined range with respect to the circumferential direction of the circle (virtual circle) along which the first to ninth driving wires (W11 to W33) are aligned.

In the present embodiment, when viewed in the attaching and detaching direction DE, the first to ninth driving wires (W11 to W33) are arranged on the outer side of the key shaft 15 so as to surround the key shaft 15. In other words, the key shaft 15 is arranged on the inner side of a circle (virtual circle) along which the first to ninth driving wires (W11 to W33) are aligned. Therefore, the key shaft 15 and the first to ninth driving wires (W11 to W33) may be arranged in a space-saving manner.

In the present embodiment, the catheter unit 100 is equipped with the operation portion 400. The operation portion 400 is configured movable (rotatable) with respect to the proximal end cover 16 and the bending driving portion 13. The operation portion 400 is rotatable about a rotation shaft 400r. The rotation shaft 400r of the operation portion 400 extends toward the attaching and detaching direction DE.

In a state where the catheter unit 100 is attached to the base unit 200, the operation portion 400 is configured movable (rotatable) with respect to the base unit 200. More specifically, the operation portion 400 is configured movably (rotatably) with respect to the base casing 200f, the wire driving portion 300, and the coupling device 21 described later.

### <Base Unit>

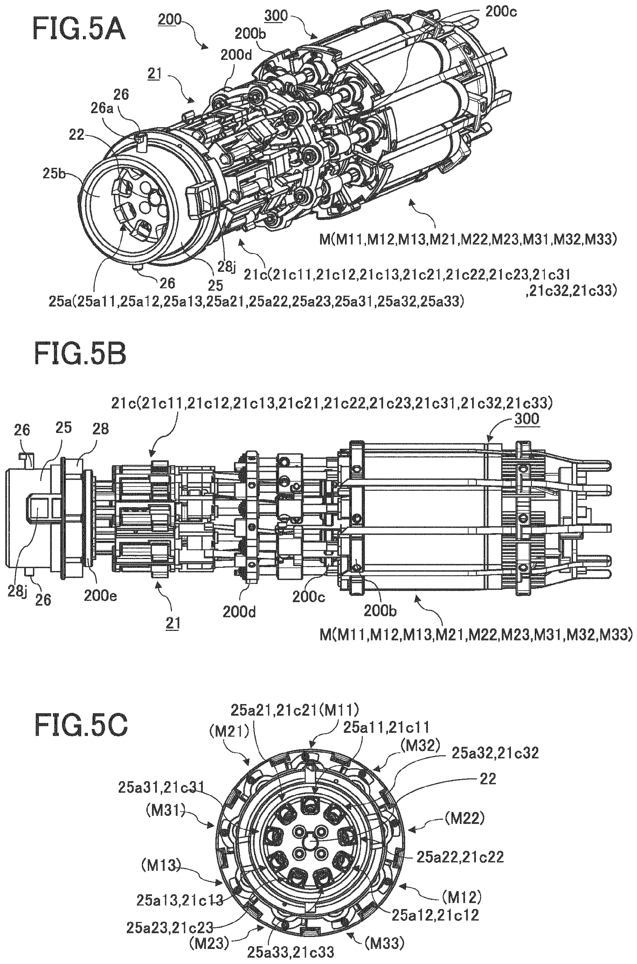

The base unit 200 and the wire driving portion 300 will be described with reference to FIGs. 5A to 5C. FIGs. 5A to 5C are explanatory views of the base unit 200 and the wire driving portion 300. FIG. 5A is a perspective view illustrating an internal configuration of the base unit 200. FIG. 5B is a side view illustrating the internal configuration of the base unit 200. FIG. 5C is a view illustrating the base unit 200 along the attaching and detaching direction DE.

As described above, the medical apparatus 1 includes the base unit 200 and the wire driving portion 300. In the present embodiment, the wire driving portion 300 is stored in the base casing 200f, and provided on the inner side of the base unit 200. In other words, the base unit 200 is equipped with the wire driving portion 300.

The wire driving portion 300 includes a plurality of driving sources (motors, actuators). In the present embodiment, the wire driving portion 300 is equipped with a first driving source M11, a second driving source M12, a third driving source M13, a fourth driving source M21, a fifth driving source M22, a sixth driving source M23, a seventh driving source M31, an eighth driving source M32, and a ninth driving source M33.

An arbitrary one among the first to ninth driving sources (M11 to M33) may be referred to as a driving source M. In the present embodiment, the first to ninth driving sources (M11 to M33) each have the same configuration.

The base unit 200 is equipped with the coupling device 21. The coupling device 21 is stored in the base casing 200f. The coupling device 21 is connected to the wire driving portion 300. The coupling device 21 includes a plurality of coupling portions. In the present embodiment, the coupling device 21 is equipped with a first coupling portion 21c11, a second coupling portion 21c12, a third coupling portion 21c13, a fourth coupling portion 21c21, a fifth coupling portion 21c22, a sixth coupling portion 21c23, a seventh coupling portion 21c31, an eighth coupling portion 21c32, and a ninth coupling portion 21c33.

An arbitrary one among the first to ninth coupling portions (21c11 to 21c33) may be called a coupling portion 21c. In the present embodiment, the first to ninth coupling portions (21c11 to 21c33) each have the same configuration.

The plurality of coupling portions are each connected to each of the plurality of driving sources, and are driven by each of the plurality of driving sources. Specifically, the first coupling portion 21c11 is connected to the first driving source M11 and driven by the first driving source M11. The second coupling portion 21c12 is connected to the second driving source M12 and driven by the second driving source M12. The third coupling portion 21c13 is connected to the third driving source M13 and driven by the third driving source M13. The fourth coupling portion 21c21 is connected to the fourth driving source M21 and driven by the fourth driving source M21. The fifth coupling portion 21c22 is connected to the fifth driving source M22 and driven by the fifth driving source M22. The sixth coupling portion 21c23 is connected to the sixth driving source M23 and driven by the sixth driving source M23. The seventh coupling portion 21c31 is connected to the seventh driving source M31 and driven by the seventh driving source M31. The eighth coupling portion 21c32 is connected to the eighth driving source M32 and driven by the eighth driving source M32. The ninth coupling portion 21c33 is connected to the ninth driving source M33 and driven by the ninth driving source M33.

As described later, the bending driving portion 13 including the first to ninth driving wires (W11 to W33) are coupled to the coupling device 21. The bending driving portion 13 receives driving force of the wire driving portion 300 through the coupling device 21 and bends the bending portion 12.

The driving wire W is coupled to the coupling portion 21c through a retained portion Wa. Each of the plurality of driving wires is coupled to each of the plurality of coupling portions. Specifically, the first retained portion Wa11 of the first driving wire W11 is coupled to the first coupling portion 21c11. The second retained portion Wa12 of the second driving wire W12 is coupled to the second coupling portion 21c12. The third retained portion Wa13 of the third driving wire W13 is coupled to the third coupling portion 21c13. The fourth retained portion Wa21 of the fourth driving wire W21 is coupled to the fourth coupling portion 21c21. The fifth retained portion Wa22 of the fifth driving wire W22 is coupled to the fifth coupling portion 21c22. The sixth retained portion Wa23 of the sixth driving wire W23 is coupled to the sixth coupling portion 21c23. The seventh retained portion Wa31 of the seventh driving wire W31 is coupled to the seventh coupling portion 21c31. The eighth retained portion Wa32 of the eighth driving wire W32 is coupled to the eighth coupling portion 21c32. The ninth retained portion Wa33 of the ninth driving wire W33 is coupled to the ninth coupling portion 21c33.

The base unit 200 includes a base frame 25. The base frame 25 is equipped with a plurality of insertion holes for passing each of the first to ninth driving wires (W11 to W33). The base frame 25 is equipped with a first insertion hole 25a11, a second insertion hole 25a12, a third insertion hole 25a13, a fourth insertion hole 25a21, a fifth insertion hole 25a22, a sixth insertion hole 25a23, a seventh insertion hole 25a31, an eighth insertion hole 25a32, and a ninth insertion hole 25a33. Each of the first to ninth insertion holes (25a11 to 25a33) corresponds to each of the first to ninth driving wires (W11 to W33). The numeral after the reference 25a indicates the numeral of the corresponding driving wire. For example, the first driving wire W11 is inserted to the first insertion hole 25a11.

An arbitrary one of the first to ninth insertion holes (25a11 to 25a33) may be referred to as an insertion hole 25a. In the present embodiment, the first to ninth insertion holes (25a11 to 25a33) each adopt the same shape.

The base frame 25 is provided with an attachment opening 25b through which the wire cover 14 is inserted. First to ninth insertion holes (25a11 to 25a33) are arranged on the bottom portion of the attachment opening 25b.

Further, the base unit 200 is equipped with a motor frame 200b, a first bearing frame 200c, a second bearing frame 200d, and a third bearing frame 200e. The motor frame 200b, the first bearing frame 200c, the second bearing frame 200d, and the third bearing frame 200e are coupled.

The base frame 25 includes the key receiving portion (key hole, base-side key, body-side key) 22 for receiving the key shaft 15. By having the key shaft 15 engage with the key receiving portion 22, it is possible to prevent the catheter unit 100 from being attached to the base unit 200 at a wrong phase.

By having the key shaft 15 engage with the key receiving portion 22, the movement of the catheter unit 100 with respect to the base unit 200 is limited within the predetermined range regarding the circumferential direction of the circle (virtual circle) along which the first to ninth driving wires (W11 to W33) are aligned.

As a result, each of the first to ninth driving wires (W11 to W33) is engaged to each of the corresponding first to ninth insertion holes (25a11 to 25a33) and to each of the corresponding first to ninth coupling portions (21c11 to 21c33). In other words, the driving wire W is prevented from being engaged with the insertion hole 25a that differs from the corresponding insertion hole 25a, and with 21c that differs from the corresponding coupling portion 21c.

The user may engage the key shaft 15 with the key receiving portion 22 to correctly couple each of the first to ninth driving wires (W 11 to W33) to each of the first to ninth coupling portions (21c11 to 21c33). Therefore, the user may easily attach the catheter unit 100 to the base unit 200.

In the present embodiment, the key shaft 15 includes a projected portion that protrudes toward a direction intersecting the attaching and detaching direction DE, and the key receiving portion 22 is equipped with a recess portion to which the projected portion is inserted. In the circumferential direction, the position at which the projected portion engages with the recess portion is the position at which the driving wire W engages with the insertion hole 25a and the coupling portion 21c corresponding thereto.

The key shaft 15 may be disposed on either one of the base unit 200 and the catheter unit 100, and the key receiving portion 22 may be disposed on the other. For example, the key shaft 15 may be arranged on the base unit 200 side, and the key receiving portion 22 may be arranged on the catheter unit 100 side.

### <Coupling of Motor and Driving Wire>

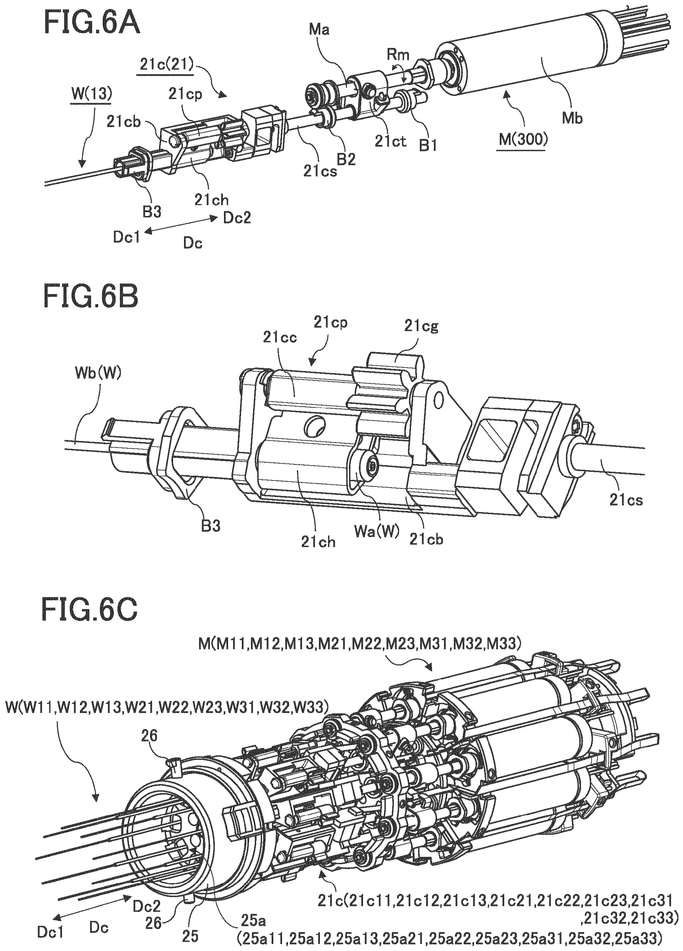

The coupling of the wire driving portion 300, the coupling device 21, and the bending driving portion 13 will be described with reference to FIGs. 6A to 6C. FIGs. 6A to 6C are explanatory views of the wire driving portion 300, the coupling device 21, and the bending driving portion 13. FIG. 6A is a perspective view of the driving source M, the coupling portion 21c, and the driving wire W. FIG. 6B is an enlarged view of the coupling portion 21c and the driving wire W. FIG. 6C is a perspective view illustrating the coupling of the wire driving portion 300, the coupling device 21, and the bending driving portion 13.

In the present embodiment, the configurations in which each of the first to ninth driving wires (W11 to W33) is coupled to each of the first to ninth coupling portions (21c11 to 21c33) are the same. Further, the configurations in which each of the first to ninth coupling portions (21c11 to 21c33) is coupled to each of the first to ninth driving sources (M11 to M33) are the same. Accordingly, in the following description, the configuration in which one driving wire W, one coupling portion 21c, and one driving source M are connected will be described.

As illustrated in FIG. 6A, the driving source M includes an output shaft Ma, and a motor body Mb that rotates the output shaft Ma in a direction of rotation Rm. A spiral groove is provided on the surface of the output shaft Ma. The output shaft Ma has a so-called screw shape. The motor body Mb is fixed to the motor frame 200b.

The coupling portion 21c includes a tractor 21ct connected to the output shaft Ma, and a tractor support shaft 21cs that supports the tractor 21ct. The tractor support shaft 21cs is connected to a coupling base 21cb.

The coupling portion 21c has a leaf spring 21ch serving as a retaining portion for retaining the retained portion Wa of the driving wire W. The driving wire W is passed through the insertion hole 25a and engaged with the coupling portion 21c. More specifically, the retained portion Wa is engaged with the leaf spring 21ch. As described later, the leaf spring 21ch may take a state (fixing state) in which the leaf spring 21ch retains the retained portion Wa by nipping and fixing the same and a state (releasing state) where the retaining of the retained portion Wa is released.

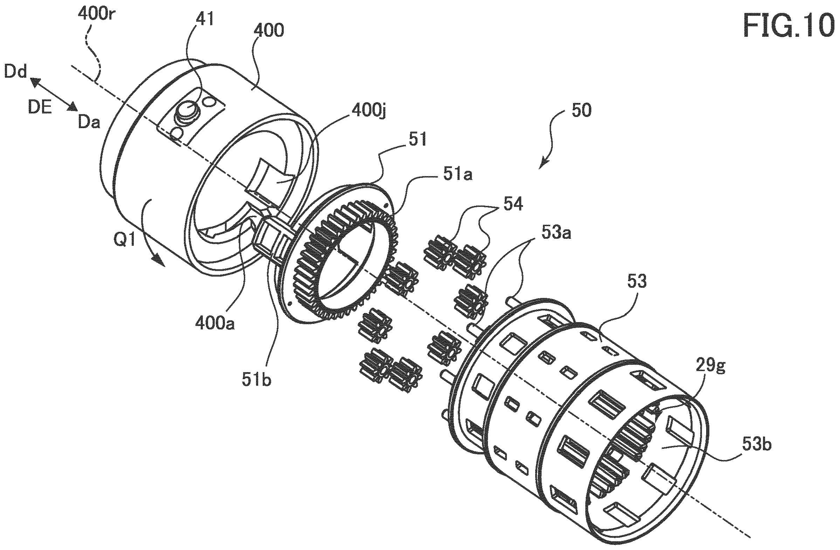

The coupling portion 21c has a pressing member 21cp serving as a switching portion. The pressing member 21cp includes a gear portion 21cg serving as a cam gear that meshes with a teeth portion 29g (refer to FIG. 10) of a planetary carrier 53 described later, and a cam 21cc serving as a cam portion (pressing portion) for pressing the leaf spring 21ch. The cam 21cc rotates integrally with the gear portion 21cg.

As described later, the cam 21cc may move with respect to the leaf spring 21ch. By the movement of the cam 21cc, the fixing state (retaining state) and a releasing state of the leaf spring 21ch may be switched.

The coupling portion 21c is supported by a first bearing B1, a second bearing B2, and a third bearing B3. The first bearing B1 is supported on the first bearing frame 200c of the base unit 200. The second bearing B2 is supported on the second bearing frame 200d of the base unit 200. The third bearing B3 is supported on the third bearing frame 200e of the base unit 200. Therefore, in a state where the output shaft Ma rotates in the direction of rotation Rm, the coupling portion 21c is regulated from rotating about the output shaft Ma. The first bearing B 1, the second bearing B2, and the third bearing B3 are disposed on each of the first to ninth coupling portions (21c11 to 21c33).

Since the rotation of the coupling portion 21c about the output shaft Ma is regulated, when the output shaft Ma is rotated, a force along the rotation shaft direction of the output shaft Ma acts on the tractor 21ct by the spiral groove on the output shaft Ma. As a result, the coupling portion 21c moves along a rotation axis direction (Dc direction) of the output shaft Ma. By the rotation of the coupling portion 21c, the driving wire W moves and the bending portion 12 bends. In this state, by switching the direction of rotation of the driving source M, the coupling portion 21c may drive the driving wire W to both the direction pressing the driving wire W and the direction pulling the driving wire W.

That is, the output shaft Ma and the tractor 21ct constitute a so-called feed screw that converts a rotary motion transmitted from the driving source M into a linear motion by a screw. In the present embodiment, the output shaft Ma and the tractor 21ct are a sliding screw, but they may also be a ball screw.

As illustrated in FIG. 6C, by attaching the catheter unit 100 to the base unit 200, each of the first to ninth driving wires (W11 to W33) is coupled to each of the first to ninth coupling portions (21c11 to 21c33).

The control portion 3 may control each of the first to ninth driving sources (M11 to M33) independently with respect to each other. That is, an arbitrary driving source among the first to ninth driving sources (M11 to M33) may be operated or stopped independently, regardless of whether the other driving sources are stopped. In other words, the control portion 3 may control each of the first to ninth driving wires (W11 to W33) independently with respect to each other. As a result, each of the first to third guide rings (J1 to J3) may be controlled independently from each other, and the bending region 12b of the bending portion 12 may be bent in an arbitrary direction.

### <Attachment of Catheter Unit>

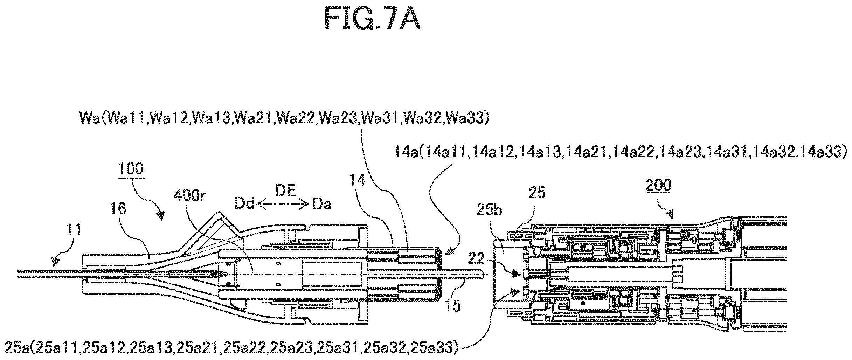

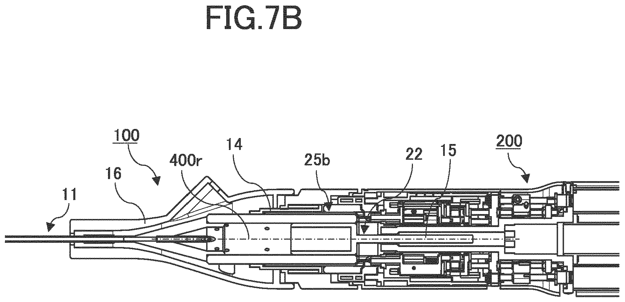

An action of attaching the catheter unit 100 to the base unit 200 will be described with reference to FIGs. 7A and 7B. FIGs. 7A and 7B are explanatory views of attachment of the catheter unit 100. FIG. 7A is a view prior to attachment of the catheter unit 100 to the base unit 200. FIG. 7B is a view after attachment of the catheter unit 100 to the base unit 200.

In the present embodiment, the attaching and detaching direction DE of the catheter unit 100 is the same as the direction of the rotation shaft 400r of the operation portion 400. In the attaching and detaching direction DE, the direction attaching the catheter unit 100 to the base unit 200 is called an attachment direction Da. In the attaching and detaching direction DE, the direction removing the catheter unit 100 from the base unit 200 (direction opposite to the attaching direction Da) is called a removal direction Dd.

As illustrated in FIG. 7A, in a state prior to attaching the catheter unit 100 to the base unit 200, the wire cover 14 is positioned at the cover position. In this state, the wire cover 14 covers the first to ninth driving wires (W11 to W33) such that the first to ninth retained portions (Wa11 to Wa33) do not protrude through the first to ninth exposing holes (14a11 to 14a33) of the wire cover 14. Therefore, in a state prior to having the catheter unit 100 attached to the base unit 200, the first to ninth driving wires (W11 to W33) may be protected.

When attaching the base unit 200 to the catheter unit 100, the key shaft 15 is engaged with the key receiving portion 22. The key shaft 15 is protruded from the wire cover 14. In the present embodiment, in a state where the key shaft 15 has reached an entrance of the key receiving portion 22, the wire cover 14 is not engaged with the attachment opening 25b. That is, if the phase of the catheter unit 100 with respect to the base unit 200 is at a phase in which the key shaft 15 and the key receiving portion 22 may not be engaged, the wire cover 14 is not engaged with the attachment opening 25b, but retained in a state position at the cover position. Therefore, even in a case where the catheter unit 100 is moved so that the key shaft 15 and the key receiving portion 22 are engaged, the first to ninth driving wires (W11 to W33) are protected.

When the key shaft 15 and the key receiving portion 22 are engaged and the catheter unit 100 is moved in the attaching direction Da with respect to the base unit 200, the catheter unit 100 is attached to the base unit 200. By attaching the catheter unit 100 to the base unit 200, the wire cover 14 moves to the exposure position. In the present embodiment, the wire cover 14 moves from the cover position to the exposure position by abutting against the base frame 25 (refer to FIG. 7B).

More specifically, when attaching the catheter unit 100, the wire cover 14 abuts against the base frame 25 and stops. In this state, by moving the catheter unit 100 in the attaching direction Da, the wire cover 14 relatively moves with respect to parts other than the wire cover 14 in the catheter unit 100. As a result, the wire cover 14 moves from the cover position to the exposure position.

While the wire cover 14 moves from the cover position to the exposure position, the retained portion Wa of the driving wire W protrudes through the exposing hole 14a of the wire cover 14 and is inserted to the insertion hole 25a. Then, the retained portion Wa is engaged with the leaf spring 21ch of the coupling portion 21c (refer to FIG. 6B).

In a state where the catheter unit 100 is simply attached to the base unit 200, the catheter unit 100 may be moved in the removal direction Dd with respect to the base unit 200 and the catheter unit 100 may be removed. Further, as described later, in a state where the catheter unit 100 is simply attached to the base unit 200, the fixing of the driving wire W and the coupling portion 21c is in a released state.

By operating the operation portion 400 in a state where the catheter unit 100 is attached to the base unit 200, the catheter unit 100 may be prevented from being removed from the base unit 200. Further, by operating the operation portion 400 in a state where the catheter unit 100 is attached to the base unit 200, the bending driving portion 13 is fixed to the coupling device 21, and the bending driving portion 13 is coupled through the coupling device 21 to the wire driving portion 300.

### <Planetary Gear Mechanism>

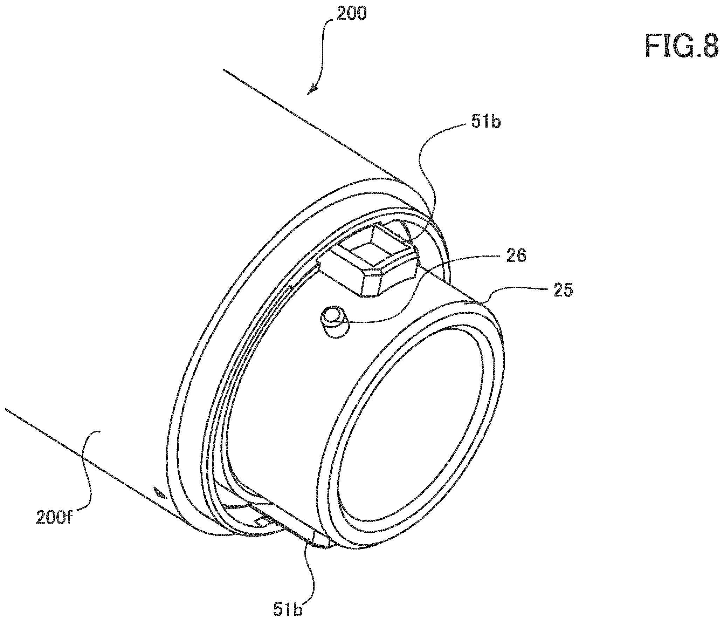

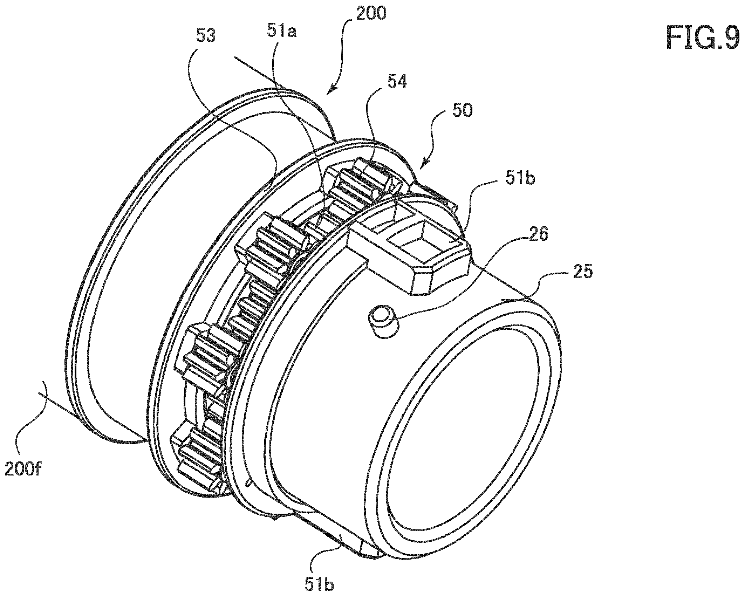

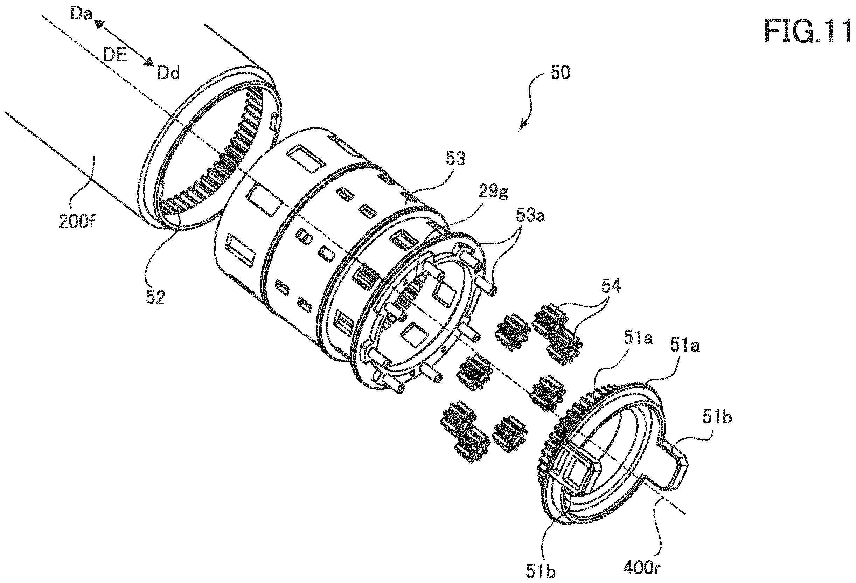

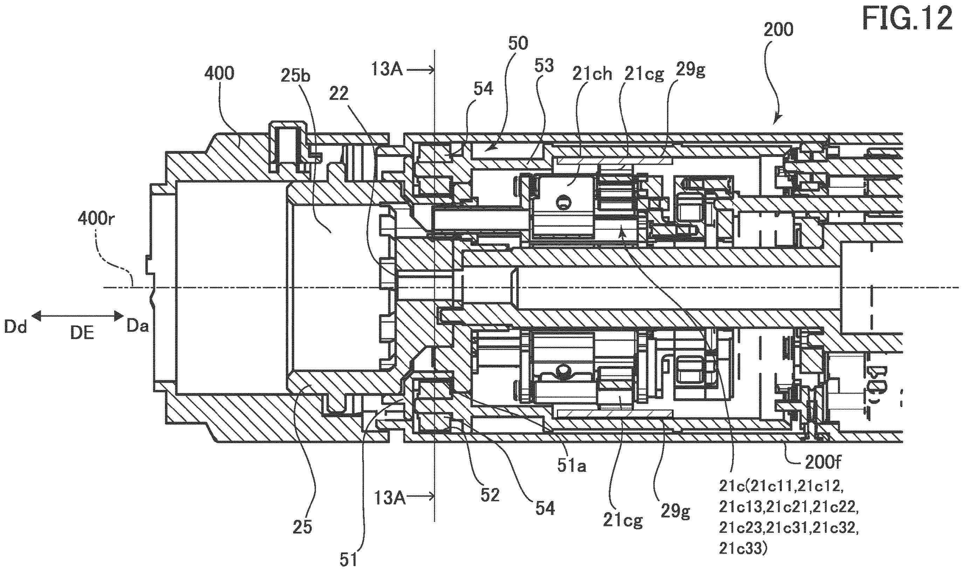

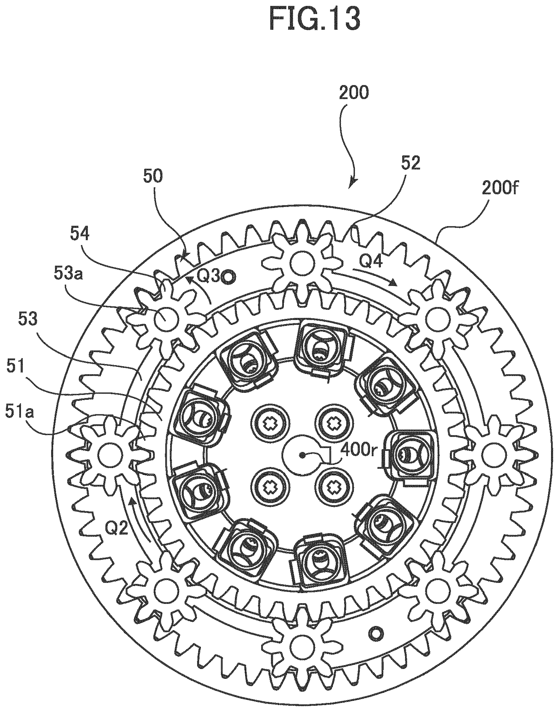

Next, a planetary gear mechanism 50 disposed on the base unit 200 will be described with reference to FIGs. 8 to 13. FIG. 8 is a perspective view illustrating a connecting part of the base unit 200 to the catheter unit 100. FIG. 9 is a perspective view illustrating the planetary gear mechanism 50 disposed in the base unit 200. In FIG. 9, a part of the base casing 200f being illustrated is cutout. FIG. 10 is an exploded perspective view illustrating the operation portion 400 and the planetary gear mechanism 50. FIG. 11 is an exploded perspective view illustrating the planetary gear mechanism 50. FIG. 12 is a cross-sectional view of the operation portion 400 and the base unit 200 cut along the rotation shaft 400r of the operation portion 400. FIG. 13 is a cross-sectional view illustrating a 13A-13A cross-section of FIG. 12.

As illustrated in FIGs. 8 to 12, the base unit 200 includes the planetary gear mechanism 50 arranged in the vicinity of the base frame 25. The planetary gear mechanism 50 includes a sun gear 51, an internal gear 52, the planetary carrier 53, and a plurality of (eight according to the present embodiment) planetary gears 54. The sun gear 51 and the planetary carrier 53 are configured rotatably about the rotation shaft 400r of the operation portion 400 in a state where the catheter unit 100 is attached to the base unit 200. The sun gear 51 and the planetary carrier 53 are supported rotatably on the base frame 25 and the base casing 200f, for example, but the configuration is not limited thereto, and they may be supported on other members of the base unit 200.

The sun gear 51 includes a gear portion 51a, and one pair of projected portions 51b and 51b engageable with one pair of engagement portions 400j disposed on the operation portion 400. The projected portions 51b and 51b extend along the attaching and detaching direction DE parallel to the axial direction of the rotation shaft 400r. As illustrated in FIGs. 8 and 9, the planetary gear mechanism 50 is mostly covered by the base casing 200f, and only the projected portions 51b and 51b of the sun gear 51 are exposed to the exterior. Therefore, it becomes possible to suppress the possibility of the user touching the gear portions of the planetary gear mechanism 50 and dust and contaminants entering the planetary gear mechanism 50, such that the damage to the planetary gear mechanism 50 may be reduced. Further, the safety of the user may be enhanced.

The internal gear 52 is disposed integrally on an inner circumference surface of the base casing 200f (refer to FIG. 11) and fixed to the base casing 200f. The planetary carrier 53 includes a plurality of (eight in the present embodiment) shaft portions 53a that rotatably support the plurality of planetary gears 54, and a plurality of teeth portions 29g that are formed on an inner circumference surface 53b. The plurality of shaft portions 53a each extend in parallel with the rotation shaft 400r. The plurality of teeth portions 29g serving as an output gear are arranged downstream of the sun gear 51, the internal gear 52, and the planetary gears 54 in the attachment direction Da in the attaching and detaching direction DE.

When the catheter unit 100 is attached to the base unit 200, a projected portion 51b of the sun gear 51 is engaged with the engagement portion 400j of the operation portion 400. Thereby, when the operation portion 400 is rotated, the rotation of the operation portion 400 is transmitted to the sun gear 51. The sun gear 51 is rotated concentrically and integrally with the operation portion 400.

As illustrated in FIGs. 12 and 13, the gear portion 51a of the sun gear 51 meshes with each of the planetary gears 54, and each of the planetary gears 54 meshes with the internal gear 52 fixed to the base casing 200f. Therefore, the rotation of the sun gear 51 is decelerated and output through the planetary gears 54 to the planetary carrier 53. That is, the sun gear 51 is an input member (input portion) configured to rotate in response to input of the rotation of the operation portion 400, the internal gear 52 is a fixed member, and the planetary carrier 53 is an output member (output portion) that outputs the transmitted rotation to the pressing member 21cp. The force that the user applies to the operation portion 400 is received by the sun gear 51, and is transmitted from the sun gear 51 through the planetary gears 54 to the planetary carrier 53. The internal gear 52 is fixed to the base unit 200 so that the position thereof within the base unit 200 is not changed. Further according to the present embodiment, eight planetary gears 54 are disposed, such that the load is shared among the eight planetary gears 54, and the planetary gear mechanism 50 may be downsized and may have a longer life.

For example, when the operation portion 400 is rotated in the arrow Q1 direction (refer to FIG. 10) by the user, the sun gear 51 also rotates in the arrow Q2 direction, as illustrated in FIG. 13. Then, the planetary gears 54 that mesh with the sun gear 51 and the internal gear 52 rotate (revolve) in the arrow Q4 direction about the rotation shaft 400r while rotating (autorotating) in the arrow Q3 direction about the shaft portions 53a. Thereby, the planetary carrier 53 that supports the planetary gears 54 also rotates in the arrow Q4 direction about the rotation shaft 400r. The arrow Q1, Q2, and Q4 directions are each the same directions of rotation about the rotation shaft 400r. The arrow Q3 direction is the opposite direction of rotation as the arrow Q1, Q2, and Q4 directions.

As described, the planetary carrier 53 rotates in an interlocked manner with the operation portion 400, and the planetary gear mechanism 50 decelerates the rotation of the operation portion 400 operated by the user and transmits the same to the planetary carrier 53. In other words, when the operation portion 400 is rotated for a first rotation angle, the planetary gear mechanism 50 decelerates the rotation transmitted from the operation portion 400 such that the planetary carrier 53 rotates for a second rotation angle that is smaller than the first rotation angle. More specifically, the rotation of the operation portion 400 is transmitted with a same rotation to the sun gear 51, and the rotation of the sun gear 51 is transmitted with a predetermined deceleration ratio to the planetary carrier 53. The deceleration ratio is calculated by (z1 + z2) / z1, wherein the number of teeth of the gear portion 51a and the internal gear 52 of the sun gear 51 are z1 and z2, respectively.

### <Fixing and Releasing of Fixing of Bending Driving Portion>

Next, a configuration for fixing the bending driving portion 13 to the coupling device 21 and a configuration for releasing the fixing of the bending driving portion 13 by the coupling device 21 will be described with reference to FIGs. 14A, 14B, 15, 16, 17, 18, and 19.

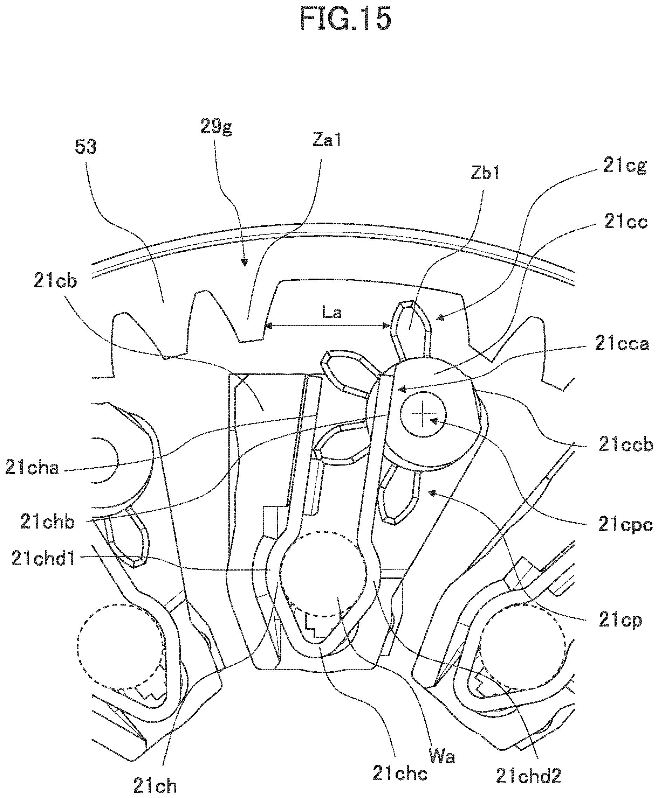

FIGs. 14A and 14B are views illustrating coupling of the catheter unit 100 and the base unit 200. FIG. 14A is a cross-sectional view of the catheter unit 100 and the base unit 200 cut along the rotation shaft 400r. FIG. 14B is a cross-sectional view of the base unit 200 cut in a direction orthogonal to the rotation shaft 400r at the area of the coupling portion 21c. FIGs. 15, 16, 17, 18, and 19 are explanatory views illustrating the fixing of the driving wire W by the coupling portion 21c.

As described above, the planetary gear mechanism 50 decelerates and transmits the rotation of the operation portion 400 operated by the user to the planetary carrier 53. As illustrated in FIGs. 14A, 14B, and 15, the teeth portion 29g disposed on the inner circumference surface 53b of the planetary carrier 53 meshes with the gear portion 21cg of the pressing member 21cp. By the gear portion 21cg being rotated by the teeth portion 29g, the cam 21cc of the pressing member 21cp presses the leaf spring 21ch, and the leaf spring 21ch (the coupling portion 21c) is switched between the fixing state and the releasing state.

More specifically, the plurality of teeth portions 29g provided on the planetary carrier 53 has a function of switching between a state where each of the first to ninth coupling portions (21c11 to 21c33) fixes each of the first to ninth driving wires (W1 1 to W33) and a state where each disengages each of the first to ninth driving wires (W11 to W33). Each of the plurality of teeth portions (operation portions, switching gear portions) 29g disposed on the planetary carrier 53 engages with the gear portion 21cg of the pressing member 21cp provided on each of the first to ninth coupling portions (21c11 to 21c33).

Specifically, the plurality of teeth portions disposed on the planetary carrier 53 in the present embodiment are equipped with a first teeth portion 29g11, a second teeth portion 29g12, a third teeth portion 29g13, a fourth teeth portion 29g21, a fifth teeth portion 29g22, a sixth teeth portion 29g23, a seventh teeth portion 29g31, an eighth teeth portion 29g32, and a ninth teeth portion 29g33. Each of the first to ninth teeth portions (29g11 to 29g33) is formed with a gap provided therebetween.

The first teeth portion 29g11 meshes with the gear portion 21cg of the first coupling portion 21c11. The second teeth portion 29g12 meshes with the gear portion 21cg of the second coupling portion 21c12. The third teeth portion 29g13 meshes with the gear portion 21cg of the third coupling portion 21c13. The fourth teeth portion 29g21 meshes with the gear portion 21cg of the fourth coupling portion 21c21. The fifth teeth portion 29g22 meshes with the gear portion 21cg of the fifth coupling portion 21c22. The sixth teeth portion 29g23 meshes with the gear portion 21cg of the sixth coupling portion 21c23. The seventh teeth portion 29g31 meshes with the gear portion 21cg of the seventh coupling portion 21c31. The eighth teeth portion 29g32 meshes with the gear portion 21cg of the eighth coupling portion 21c32. The ninth teeth portion 29g33 meshes with the gear portion 21cg of the ninth coupling portion 21c33.

An arbitrary one among the first to ninth teeth portions (29g11 to 29g33) may be called the teeth portion 29g. In the present embodiment, the first to ninth teeth portions (29g11 to 29g33) each have the same configuration.

In the present embodiment, the configurations in which each of the first to ninth driving wires (W11 to W33) and each of the first to ninth coupling portions (21c11 to 21c33) are coupled are the same. Further, the configurations in which each of the first to ninth coupling portions (21c11 to 21c33) and each of the first to ninth teeth portions (29g11 to 29g33) are connected are the same. Therefore, in the following description, one driving wire W, one coupling portion 21c, and one teeth portion 29g are used to describe a configuration in which they are connected.

In each of the first to ninth coupling portions (21c11 to 21c33), the pressing member 21cp rotates by the gear portion 21cg being moved by the teeth portion 29g, and the cam 21cc moves to a pressing position and to a retracting position being retracted from the pressing position.

By rotating the operation portion 400, the planetary carrier 53 rotates through the sun gear 51 and the planetary gears 54 of the planetary gear mechanism 50. By the rotation of the planetary carrier 53, each of the first to ninth coupling portions (21c11 to 21c33) are operated. That is, by the action of rotating one operation portion 400, the first to ninth coupling portions (21c11 to 21c33) may be operated.

The operation portion 400 may be moved between the fixed position (locked position) and the removal position in a state where the catheter unit 100 is attached to the base unit 200. Further, as described later, the operation portion 400 may be moved to the release position in a state where the catheter unit 100 is attached to the base unit 200. In the circumferential direction of the operation portion 400, the release position is positioned between the fixed position and the removal position. In a state where the operation portion 400 is positioned at the removal position, the catheter unit 100 is attached to the base unit 200.

In a state where the catheter unit 100 is attached to the base unit 200, the fixing (locking) of the driving wire W to the coupling portion 21c is in a released state. This state is called a releasing state of the coupling portion 21c. A state where the driving wire W is fixed (locked) to the coupling portion 21c is called a fixing state of the coupling portion 21c. That is, the state of the coupling portion 21c when the leaf spring 21ch is in the fixing state (retaining state) is called the fixing state (retaining state), and the state of the coupling portion 21c when the leaf spring 21ch is in the releasing state is called the releasing state.

An action of fixing the driving wire W to the coupling portion 21c will be described with reference to FIGs. 15, 16, 17, 18, and 19. In the present embodiment, each teeth portion 29g of the planetary carrier 53 includes three teeth Za1, Za2, and Za3, and the gear portion 21cg of each of the pressing members 21cp respectively include four teeth Zb1, Zb2, Zb3, and Zb4.

In a state after the catheter unit 100 has been attached to the base unit 200 and before the operation portion 400 is operated, the catheter unit 100 may be removed from the base unit 200. In the following description, a state where the catheter unit 100 may be removed from the base unit 200 is called a removable state.

FIG. 15 is a view illustrating the state of the planetary carrier 53 and the coupling portion 21c in a removable state. FIG. 15 is a view illustrating the planetary carrier 53 and the coupling portion 21c in a state where the operation portion 400 is in a removal position.

The leaf spring 21ch of the coupling portion 21c includes a fixed portion 21cha fixed to the coupling base 21cb, and a pressed portion 21chb that is in contact with the cam 21cc of the pressing member 21cp. The leaf spring 21ch includes a first portion 21chd1 and a second portion 21chd2. When the catheter unit 100 is attached to the base unit 200, the retained portion Wa is inserted between the first portion 21chd1 and the second portion chd2.

The cam 21cc includes a retaining surface 21cca and a pressing surface 21ccb. Regarding the radial direction of rotation of the pressing member 21cp, the retaining surface 21cca is arranged at a position close to a center of rotation 21cpc of the pressing member 21cp than the pressing surface 21ccb.

As illustrated in FIG. 15, in a removable state (in a state where the operation portion 400 is at the removal position), the leaf spring 21ch is retained at a position where the pressed portion 21chb is in contact with the retaining surface 21cca. Further, the tooth Za1 of the planetary carrier 53 and the tooth Zb1 of the gear portion 21cg are stopped in a state where a clearance La is formed therebetween.

In the direction of rotation of the operation portion 400, the direction in which the operation portion 400 moves from the removal position toward the release position and the fixed position is called a locking direction (fixing direction), and the direction in which the operation portion 400 moves from the fixed position to the release position and the removal position is called a releasing direction. The operation portion 400 rotates from the release position toward the releasing direction and moves to the removal position. The operation portion 400 rotates from the release position to the locking direction ad moves to the fixed position.

In a state where the catheter unit 100 is attached to the base unit 200 and the operation portion 400 is in the removal position, the coupling portion 21c is in the releasing state, and the fixing of the driving wire W by the coupling portion 21c is in a released state.

When the coupling portion 21c is in the releasing state, the cam 21cc is positioned at a retracting position retracted from the pressing position described later. In this state, the fixing of the retained portion Wa by the leaf spring 21ch is in a released state. A force by which the first portion 21chd1 and the second portion 21chd2 clamps the retained portion Wa when the coupling portion 21c is in the releasing state is smaller than a force by which the first portion 21chd1 and the second portion 21chd2 clamps the retained portion Wa when the coupling portion 21c is in the fixing state.

In a state where the catheter unit is moved in the removal direction Dd with respect to the base unit 200 when the coupling portion 21c is in the releasing state, the retained portion Wa may be drawn out from between the first portion 21chd1 and the second portion 21chd2.

In a state where the coupling portion 21c is in the releasing state, it is preferable that the first portion 21chd1 and the second portion 21chd2 are in a state where a force for clamping the retained portion Wa is not generated (a state where the magnitude is zero). In a state where the coupling portion 21c is in the releasing state, it is preferable that a gap is formed between at least one of the first portion 21chd1 and the second portion 21chd2 and the retained portion Wa.

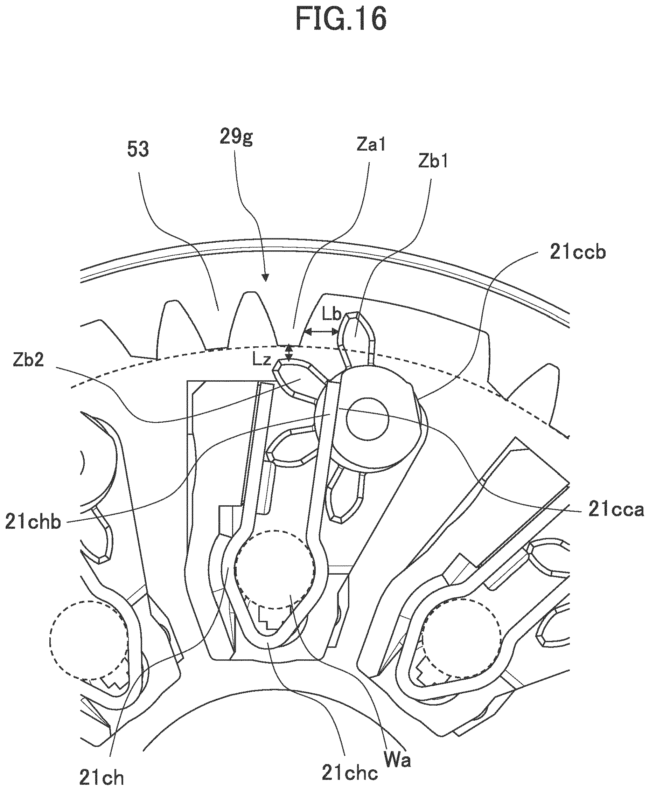

FIG. 16 illustrates a view of the state of the planetary carrier 53 and the coupling portion 21c when the operation portion 400 is rotated from the removal position to the locking direction. FIG. 16 is a view of the state of the planetary carrier 53 and the coupling portion 21c in a state where the operation portion 400 is in the release position.

When the operation portion 400 is rotated in the locking direction in a state where the operation portion 400 is in the removal position (FIG. 15), the planetary carrier 53 rotates clockwise. Then, the operation portion 400 is positioned at the release position.

Even when the operation portion 400 is rotated, the key shaft 15 and the key receiving portion 22 are engaged, such that the entirety of the catheter unit 100 (excluding the operation portion 400) is regulated from rotating with respect to the base unit 200. That is, in a state where the entirety of the catheter unit 100 (excluding the operation portion 400) and the base unit 200 are stopped, the operation portion 400 is rotatable with respect thereto.

By the planetary carrier 53 rotating clockwise, a clearance between the tooth Za1 of the planetary carrier 53 and the tooth Zb1 of the gear portion 21cg is reduced from clearance La to clearance Lb.

The tooth Zb2 of the gear portion 21cg is arranged at a position with a clearance Lz from a tooth tip circle (dotted line) of the teeth portion 29g of the planetary carrier 53. Therefore, the planetary carrier 53 is rotatable without interfering with the tooth Zb2. Meanwhile, the coupling portion 21c is maintained at a same state (releasing state) as the state illustrated in FIG. 15.

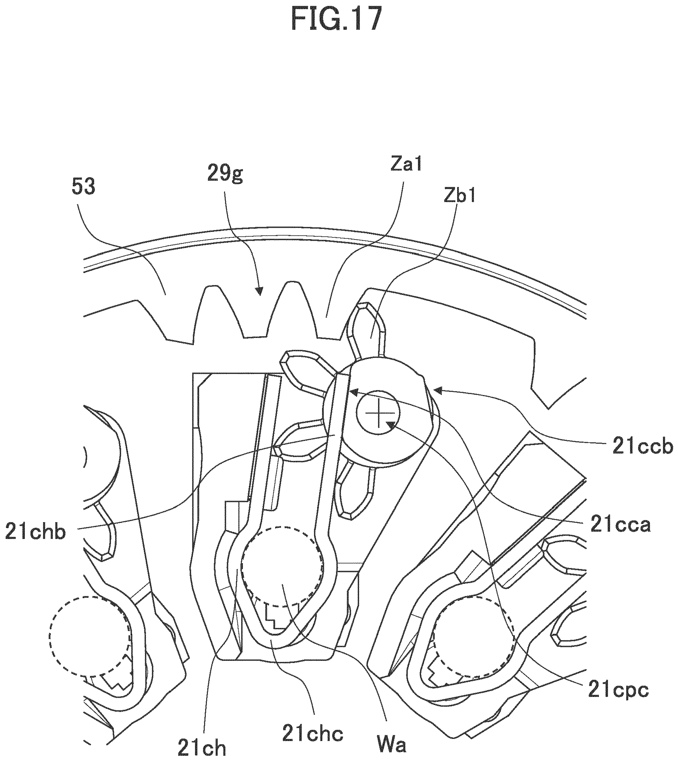

When the operation portion 400 is rotated further in the locking direction from the state illustrated in FIG. 16, the planetary carrier 53 rotates further in the clockwise direction. The state of the planetary carrier 53 and the coupling portion 21c is illustrated in FIG. 17.

FIG. 17 is a view illustrating a state of the planetary carrier 53 and the coupling portion 21c when the operation portion 400 is rotated from the release position to the locking direction. As illustrated in FIG. 17, when the operation portion 400 is rotated from the release position to the locking direction, the tooth Za1 of the planetary carrier 53 and the tooth Zb1 of the gear portion 21cg come into contact with each other. Meanwhile, the coupling portion 21c is in a same state as the state illustrated in FIGs. 15 and 16, and it is retained at the releasing state.

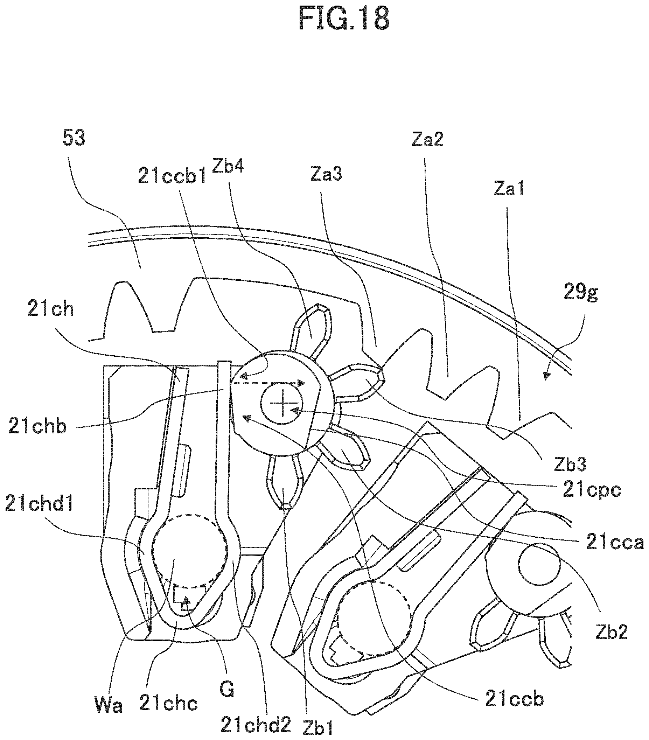

FIG. 18 is a view illustrating a state where the pressing member 21cp is rotated by the operation portion 400 being rotated in the locking direction. As illustrated in FIG. 18, in a state where the operation portion 400 is rotated further in the locking direction from the state of FIG. 17, the planetary carrier 53 rotates further in the clockwise direction.

By the planetary carrier 53 moving from the state of FIG. 17 to the state of FIG. 18, the planetary carrier 53 rotates the gear portion 21cg in the clockwise direction. When the gear portion 21cg rotates, the retaining surface 21cca separates from the pressed portion 21chb, and the pressing surface 21ccb approaches the pressed portion 21chb. Then, nipping of the retained portion Wa by the first portion 21chd1 and the second portion 21chd2 is started.

Then, the tooth Za3 of the planetary carrier 53 moves to a position separated from the tooth Zb3 of the gear portion 21cg while having the pressed portion 21chb pressed by a corner portion 21ccb1 arranged at the end portion of the pressing surface 21ccb. In this state, the retained portion Wa is in a state nipped by the first portion 21chd1 and the second portion 21chd2.

When the tooth Za3 of the planetary carrier 53 separates from the tooth Zb3 of the gear portion 21cg, transmission of driving force from the planetary carrier 53 to the gear portion 21cg is ended. In this state, the cam 21cc is in a state where the corner portion 21ccb1 receives reaction from the leaf spring 21ch.

In the radial direction of rotation of the pressing member 21cp, the reaction of the leaf spring 21ch acting on the corner portion 21ccb1 acts on a position separated from the center of rotation 21cpc of the pressing member 21cp and the pressing member 21cp rotates clockwise. In this state, the pressing member 21cp rotates in a same direction as the direction of rotation caused by the planetary carrier 53 rotating clockwise.

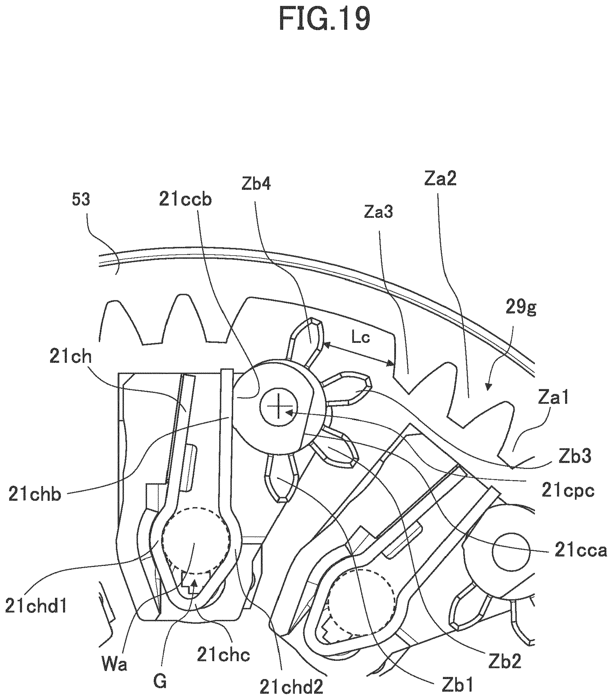

FIG. 19 illustrates a state of the planetary carrier 53 and the coupling portion 21c in a state where the operation portion 400 is in the fixed position. As illustrated in FIG. 19, the pressing member 21cp rotates further by receiving the reaction of the leaf spring 21ch from the state illustrated in FIG. 18.

As illustrated in FIG. 19, the pressing member 21cp stops in a state where the pressing surface 21ccb of the cam 21cc and the pressed portion 21chb of the leaf spring 21ch are in surface contact. That is, the surface of the pressing surface 21ccb and that of the pressed portion 21chb are in a state aligned on a same plane. In this state, the coupling portion 21c is in the fixing state. In a state where the coupling portion 21c is in the fixing state, the cam 21cc of the pressing member 21cp is positioned at the pressing position, and the pressing surface 21ccb presses the pressed portion 21chb.

When the coupling portion 21c is in the fixing state, the retained portion Wa is nipped by the first portion 21chd1 and the second portion 21chd2. That is, the leaf spring 21ch is pressed by the cam 21cc and the retained portion Wa is clamped by the leaf spring 21ch. As a result, the retained portion Wa is fixed by the leaf spring 21ch.

In the present embodiment, the first portion 21chd1 and the second portion 21chd2 of the leaf spring 21ch press the retained portion Wa at mutually separated positions. Further, a bending portion 21chc connecting the first portion 21chd1 and the second portion 21chd2 is disposed between the first portion 21chd1 and the second portion 21chd2. The bending portion 21chc is arranged with a gap G from the retained portion Wa. Thereby, the retained portion Wa may be fixed stably by the first portion 21chd1 and the second portion 21chd2.

Resin or metal may be used as the material of the leaf spring 21ch, and preferably, metal is used.

When the coupling portion 21c is in the fixing state, the retained portion Wa is restricted from being drawn out from between the first portion 21chd1 and the second portion 21chd2.

The tooth Za3 of the planetary carrier 53 and the tooth Zb4 of the gear portion 21cg are stopped at a position where a clearance Lc is formed therebetween. Further, a tooth tip surface of the tooth Za3 is inclined so as to recede from the rotational axis toward a downstream side in the releasing direction with respect to a cylindrical surface that is in contact with the tooth tip surfaces of other teeth Za1 and Za2 about the rotational axis (the rotation shaft 400r) of the planetary carrier 53. Thereby, when the planetary carrier 53 is rotated in the releasing direction from the state illustrated in FIG. 19, the tooth Za3 of the planetary carrier 53 may reach the tooth Zb4 beyond the tooth Zb3 without prying the tooth Zb3 of the gear portion 21cg.

When releasing the fixture of the driving wire W and the coupling portion 21c, the operation portion 400 positioned at the fixed position is rotated in the releasing direction. In this state, the planetary carrier 53 rotates in a counterclockwise direction from the state illustrated in FIG. 19. When the planetary carrier 53 rotates in the counterclockwise directions, the tooth Za3 of the planetary carrier 53 abuts against the tooth Zb4 of the gear portion 21cg, and the pressing member 21cp is rotated in the counterclockwise direction.

By rotating the planetary carrier 53 further in the counterclockwise direction, the fixing of the driving wire W by the coupling portion 21c is released. The action of the planetary carrier 53 and the pressing member 21cp in this state is the opposite action as the action described above. That is, the fixing of the driving wire W by the coupling portion 21c is released by an opposite action as the above-described action of fixing the driving wire W by the coupling portion 21c.

The above-described action is performed at each of the first to ninth coupling portions (21c11 to 21c33). That is, during the process in which the operation portion 400 moves from the removal position to the fixed position, the first to ninth coupling portions (21c11 to 21c33) change from the releasing state to the fixing state by the movement (rotation) of the operation portion 400. During the process in which the operation portion 400 moves from the fixed position to the removal position, the first to ninth coupling portions (21c11 to 21c33) change from the fixing state to the releasing state by the movement (rotation) of the operation portion 400. In other words, the user may switch the releasing state and the fixing state of the plurality of coupling portions by operating one operation portion 400.

That is, there is no need to provide an operation portion for switching the releasing state and the fixing state to each of the plurality of coupling portions and have the user operate the same. Therefore, the user may easily attach and detach the catheter unit 100 to and from the base unit 200. Further, the medical apparatus 1 may be simplified.

A state in which each of the first to ninth driving wires (W11 to W33) are fixed by each of the first to ninth coupling portions (21c11 to 21c33) is called a first state. A state in which the fixing of each of the first to ninth driving wires (W11 to W33) to each of the first to ninth coupling portions (21c11 to 21c33) is released is called a second state.

The first state and the second state are switched in an interlocked manner with the movement of the operation portion 400. That is, the first state and the second state are switched in an interlocked manner with the movement of the operation portion 400 between the removal position and the fixed position.

The planetary carrier 53 of the planetary gear mechanism 50 is configured to be interlocked with the operation portion 400. In the present embodiment, the sun gear 51, the internal gear 52, and the planetary gears 54 of the planetary gear mechanism 50 function as a transmission member for interlocking the operation portion 400 and the planetary carrier 53. The planetary gear mechanism 50 has a function as an interlocking portion that is interlocked with the operation portion 400 such that the first state and the second state are switched in an interlocked manner with the movement of the operation portion 400.

Specifically, the planetary carrier 53 of the planetary gear mechanism 50 moves a part of the leaf spring 21ch (the pressed portion 21chb) with respect to the retained portion Wa in an interlocked manner with the movement of the operation portion 400 in a state where the catheter unit 100 is attached to the base unit 200. By the movement of the pressed portion 21chb, the fixing state and the releasing state of the coupling portion 21c are switched.

### <Movement of Operation Portion>

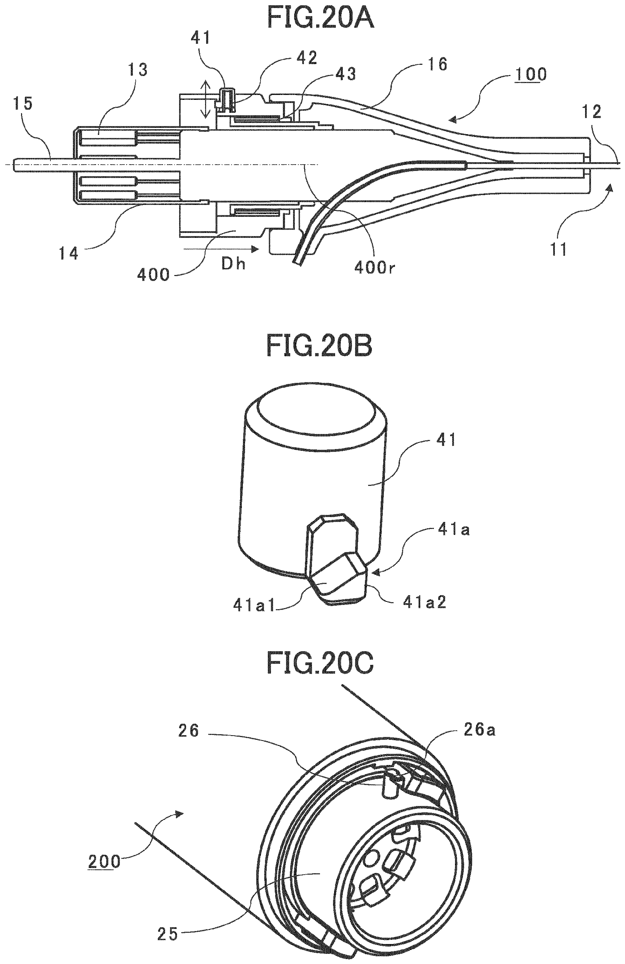

Next, the movement of the operation portion 400 will be described with reference to FIGs. 20A to 22C.

In the present embodiment, the operation portion 400 is configured to be movable between the removal position, the release position, and the fixed position in a state where the catheter unit 100 is attached to the base unit 200. The release position is positioned between the removal position and the fixed position.

In the present embodiment, the first state and the second state are switched in an interlocked manner with the movement of the operation portion 400 between the release position and the fixed position of the operation portion 400.

In the present embodiment, the operation portion 400 is movable between the removal position and the fixed position by moving in a direction that differs from the attaching and detaching direction DE. The operation portion 400 moves between the removal position and the fixed position by moving in a direction intersecting (preferably orthogonal to) the attaching and detaching direction DE. In the present embodiment, the operation portion 400 moves between the removal position and the fixed position by rotating about the rotation shaft 400r extending in the attaching and detaching direction DE. Accordingly, the operability of the user operating the operation portion 400 is desirable.

FIGs. 20A to 20C are explanatory views illustrating the catheter unit 100 and the base unit 200. FIG. 20A is a cross-sectional view of the catheter unit 100. FIG. 20B is a perspective view of a button 41. FIG. 20C is a perspective view of the base unit 200.

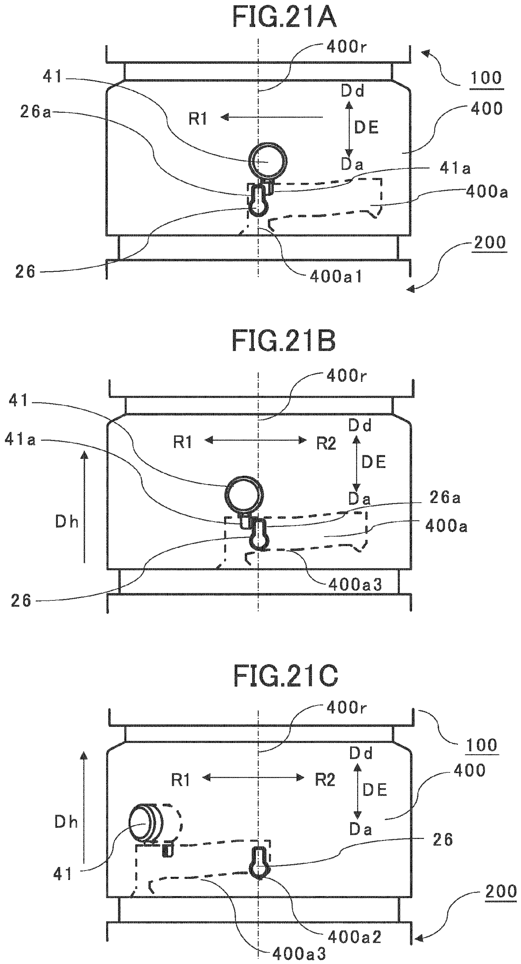

FIGs. 21A to 21C are views illustrating the action of the operation portion 400. FIG. 21A is a view illustrating a state where the operation portion 400 is in the removal position. FIG. 21B is a view illustrating a state where the operation portion 400 is in the release position. FIG. 21C is a view illustrating a state where the operation portion 400 is in the fixed position.

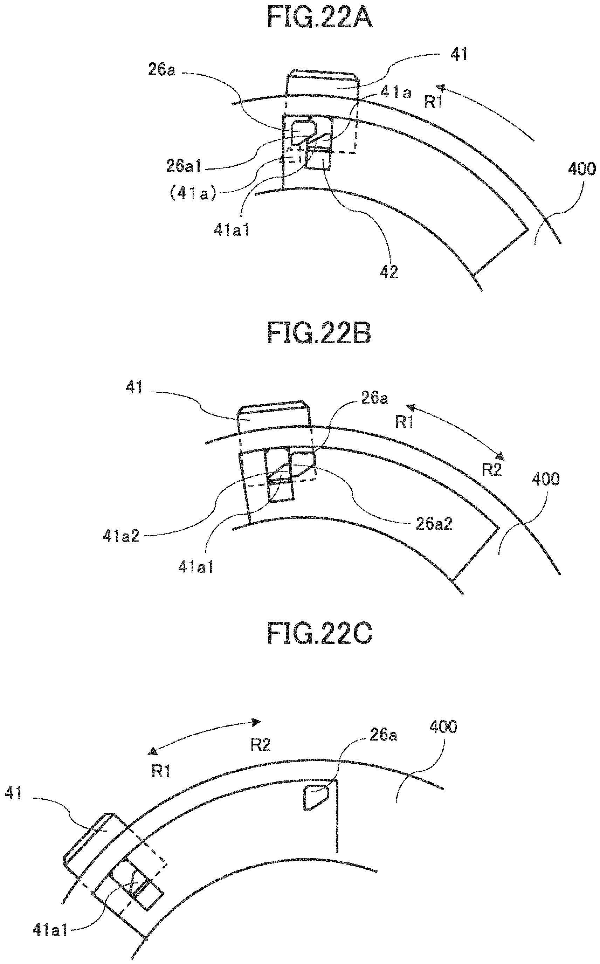

FIGs. 22A to 22C are cross-sectional views illustrating the action of the operation portion 400. FIG. 22A is a cross-sectional view illustrating a state where the operation portion 400 is in the removal position. FIG. 22B is a cross-sectional view illustrating a state where the operation portion 400 is in the release position. FIG. 22C is a cross-sectional view illustrating a state where the operation portion 400 is in the fixed position.

When the operation portion 400 is in the fixed position, the coupling portion 21c is in the fixing state, and the retained portion Wa of the driving wire W is fixed to the corresponding coupling portion 21c (refer to FIG. 19).

When the operation portion 400 is in the release position, the coupling portion 21c is in the releasing state, and the locking of the retained portion Wa of the driving wire W to the coupling portion 21c is released (refer to FIG. 16). In this state, the connection between the driving wire W and the wire driving portion 300 is disconnected. Therefore, in a state where the catheter 11 receives external force, the bending portion 12 may be bent freely without resistance being applied from the wire driving portion 300.

When the operation portion 400 is in the removal position, removal of the catheter unit 100 from the base unit 200 is allowed. Further, in a state where the operation portion 400 is in the removal position, the catheter unit 100 may be attached to the base unit 200. When the operation portion 400 is in the removal position, the coupling portion 21c is in the releasing state, and the locking of the retained portion Wa of the driving wire W to the coupling portion 21c is released (refer to FIG. 15).

As illustrated in FIG. 20A, the catheter unit 100 includes an operation portion urging spring 43 urging the operation portion 400, the button 41 serving as a moving member, and a button spring 42 urging the button 41.

In the present embodiment, the operation portion urging spring 43 is a compression spring. The operation portion 400 is urged by the operation portion urging spring 43 toward an approximating direction Dh to the proximal end cover 16.

In the present embodiment, the button 41 and the button spring 42 are disposed on the operation portion 400. When the operation portion 400 moves to the removal position, the release position, and the fixed position, the button 41 and the button spring 42 move together with the operation portion 400.

The button 41 is configured movable with respect to the operation portion 400 toward a direction intersecting the direction of the rotation shaft 400r of the operation portion 400. The button 41 is urged toward an outer side of the catheter unit 100 (direction receding from the rotation shaft 400r) by the button spring 42.

As described later, the movement of the operation portion 400 from the release position to the removal position is regulated by the button 41. Further, the movement of the operation portion 400 from the release position to the removal position is permitted by the button 41 being moved with respect to the operation portion 400.

The button 41 includes a button projection (regulated portion) 41a. The button projection 41a includes an inclined surface 41a1 and a regulated surface 41a2.

The base unit 200 is equipped with the base frame 25. The base frame 25 is equipped with a lock shaft 26. The lock shaft 26 is equipped with a lock projection (regulating portion) 26a.

In the present embodiment, a plurality of (two in the present embodiment) lock shafts 26 are provided. All the lock shafts 26 may be provided with the lock projection 26a, or some of the lock shafts 26 may be provided with the lock projection 26a.

Meanwhile, as illustrated in FIG. 10 and FIGs. 21A to 21C, a lock groove 400a that engages with the lock shaft 26 is provided on the inner side of the operation portion 400. The lock groove 400a is extended in a direction that differs from the attaching and detaching direction DE. In the present embodiment, it is extended in the direction of rotation of the operation portion 400. The lock groove 400a may be described as being extended in a direction intersecting (direction orthogonal to) the attaching and detaching direction DE.

If a plurality of lock shafts 26 are provided, the lock groove 400a is provided on each of the plurality of lock shafts 26.

As illustrated in FIG. 21A, when the catheter unit 100 is attached to the base unit 200, the lock shaft 26 is engaged with the lock groove 400a through an entrance 400a1 of the lock groove 400a.

In this state, the operation portion 400 is positioned at the removal position, and the coupling portion 21c is in the releasing state (refer to FIG. 15). Therefore, the fixing by each of the first to ninth coupling portions (21c11 to 21c33) to each of the first to ninth driving wires (W11 to W33) is in the released state. Further, as illustrated in FIG. 22A, the button projection 41a and the lock projection 26a are opposed to each other.

In a state where the operation portion 400 is in the removal position, when the operation portion 400 is rotated in a locking direction R1, the inclined surface 41a1 of the button projection 41a abuts against an inclined surface 26a1 of the lock projection 26a. The button 41 moves toward an inner side (approximating direction toward the rotation shaft 400r) of the operation portion 400 against the urging force of the button spring 42. Then, the button projection 41a moves beyond the lock projection 26a, and the operation portion 400 moves to the release position (refer to FIG. 22B).

In this state, the coupling portion 21c is in the releasing state (refer to FIG. 16). Therefore, the fixing of each of the first to ninth coupling portions (21c11 to 21c33) to each of the first to ninth driving wires (W11 to W33) is in the released state.

In the present embodiment, the movement of the operation portion 400 from the removal position to the release position is permitted even if the button 41 is not operated. That is, the user is not required to operate the button 41 when the operation portion 400 is moved from the removal position to the release position.

If the operation portion 400 is rotated in the locking direction R1 in a state where the operation portion 400 is positioned at the release position, the operation portion 400 is moved to the fixed position. In a state where the operation portion 400 is in the fixed position, a positioning portion 400a2 of the lock groove 400a is positioned at a position corresponding to the lock shaft 26. The operation portion 400 is urged to the approximating direction Dh toward the proximal end cover 16 by the operation portion urging spring 43. As a result, the positioning portion 400a2 is engaged with the lock shaft 26.

During the process in which the operation portion 400 moves from the release position to the fixed position, the retained portion Wa of the driving wire W is fixed to the coupling portion 21c as described above.

In a state where the operation portion is positioned at the fixed position, the coupling portion 21c is in the fixing state (refer to FIG. 14). Therefore, each of the first to ninth driving wires (W11 to W33) is fixed to each of the first to ninth coupling portions (21c11 to 21c33). In this state, the driving force from the wire driving portion 300 is may be transmitted to the bending driving portion 13. That is, the driving force from each of the first to ninth driving sources (M11 to M33) may be transmitted to each of the first to ninth driving wires (W11 to W33) through the first to ninth coupling portions (21c11 to 21c33).

In a state where the operation portion 400 is in the release position, a wall 400a3 that forms the lock groove 400a is positioned upstream of the lock shaft 26 in the removal direction Dd of the catheter unit 100. In a state where the operation portion 400 is in the fixed position, the positioning portion 400a2 is positioned upstream of the lock shaft 26 in the removal direction Dd. As a result, when the operation portion 400 is in the release position and in the fixed position, the catheter unit 100 is regulated from being removed from the base unit 200. Meanwhile, when the operation portion 400 is in the removal position, the entrance 400a1 of the lock groove 400a is positioned upstream of the lock shaft 26 in the removal direction Dd. As a result, the catheter unit 100 is permitted to be removed from the base unit 200.

If the operation portion 400 is rotated toward a releasing direction R2 in a state where the operation portion 400 is in the fixed position, the operation portion 400 is positioned at the release position. During the process in which the operation portion 400 is moved from the fixed position to the release position, the retained portion Wa of the driving wire W is disengaged from the coupling portion 21c, as described above.

In a state where the operation portion 400 is positioned at the release position, the regulated surface 41a2 of the button projection 41a is abutted against a regulation surface 26a2 of the lock projection 26a (refer to FIG. 22B). In this state, the operation portion 400 is regulated from being rotated in the releasing direction R2. Further, the catheter unit 100 is regulated from being removed from the base unit 200.

In a state where the operation portion 400 is positioned at the release position, when the user presses the button 41 toward the inner side of the operation portion 400, the regulated surface 41a2 is separated from the regulation surface 26a2 and the button projection 41a moves beyond the lock projection 26a. As a result, the operation portion 400 is permitted to rotate in the releasing direction R2, and the operation portion 400 may be moved from the release position to the removal position.

When the operation portion 400 is positioned at the removal position, the coupling portion 21c is in the releasing state. Therefore, when the catheter unit 100 is removed from and attached to the base unit 200, the load acting on the driving wire W (for example, the resistance received by the coupling portion 21c) may be reduced. Therefore, the user may easily attach and detach the catheter unit 100.

When the operation portion 400 is positioned at the release position, the catheter unit 100 is regulated from being removed from the base unit 200, and the coupling portion 21c is in the releasing state. When the coupling portion 21c is in the releasing state, the connection between the driving wire W and the wire driving portion 300 is disconnected, and the bending portion 12 may be bent freely without resistance being applied from the wire driving portion 300, as described above.

By positioning the operation portion 400 at the release position in a state where the catheter 11 is inserted to the inner side of the target, the user may stop the driving of the catheter 11 by the wire driving portion 300. Further, since the catheter unit 100 is regulated from being removed from the base unit 200, the user may hold the base unit 200 and draw the catheter 11 out from the inner side of the target.

According further to the configuration of the present embodiment, in a case where the button 41 is not operated, the operation portion 400 is regulated from being moved from the release position to the removal position. Therefore, when the user moves the operation portion 400 from the fixed position to the release position, it becomes possible to suppress the operation portion 400 from being moved erroneously to the removal position.

In the present embodiment, the number of the lock projection 26a and the button 41 is one each. However, the medical apparatus 1 may include a plurality of the lock projection 26a and a plurality of the button 41.

### Advantage of Present Embodiment

As described above, the rotation of the operation portion 400 is transmitted through the planetary gear mechanism 50 to the gear portion 21cg of the pressing member 21cp such that the state of the leaf spring 21ch may be switched between the fixing state and the releasing state. The leaf spring 21ch (the coupling portion 21c) is switched between the fixing state and the releasing state by the rotation of the pressing member 21cp.

According to the present embodiment, the rotation of the operation portion 400 is decelerated by the planetary gear mechanism 50 and transmitted to the gear portion 21cg of the pressing member 21cp. That is, the planetary gear mechanism 50 is a deceleration mechanism that decelerates and transmits the rotation of the operation portion 400. According to the present embodiment, the rotation angle of the planetary carrier 53 is smaller than the rotation angle of the operation portion 400 and the sun gear 51. Therefore, when operating the operation portion 400 from the removal position to the fixed position, the operation portion 400 must be rotated against the urging force of the leaf spring 21ch, but the operation force for operating the operation portion 400 by the user may be reduced, and the operability may be improved.

Further, when the gear portion 21cg of the pressing member 21cp is meshed with the teeth portion 29g of the planetary carrier 53, reaction acts on the pressing member 21cp by the elastic force when the elastically deformed leaf spring 21ch returns. This reaction is transmitted through respective gear trains of the planetary gear mechanism 50 to the operation portion 400, but since it is attenuated in the process in which the respective gear trains of the planetary gear mechanism 50 are rotated and not directly transmitted, the sense of operation of the operation portion 400 may be improved.

Apart of the base frame 25, the first to ninth coupling portions (21c11 to 21c33), the key shaft 15 and bending driving portion 13 of the catheter unit 100 etc. are accommodated within a space on the inner side in the radial direction of the planetary gear mechanism 50. Further, the sun gear 51 and the planetary carrier 53 of the planetary gear mechanism 50 are configured to rotate concentrically as the operation portion 400. Therefore, the space efficiency is good, and the increase in size of the apparatus may be suppressed even when the planetary gear mechanism 50 is disposed.

Further, by adjusting the deceleration ratio of the planetary gear mechanism 50, the rotational amount of the operation portion 400 and the operation force of the operation portion 400 by the user may be set arbitrarily. For example, the deceleration ratio of the planetary gear mechanism 50 may be set between 1.5 and 3.0. Preferably, the deceleration ratio of the planetary gear mechanism 50 is set to 2.4 such that the planetary carrier 53 is rotated for approximately 50 degrees when the operation portion 400 is rotated for approximately 120 degrees between the removal position and the fixed position. The operation for rotating the operation portion 400 for 120 degrees is an appropriate operation range that the user may perform by one action, and by further setting the deceleration ratio to approximately 2.4, no great operation force is required to transit the coupling portion 21c between the releasing state and the fixing state.

### (Modified Example)

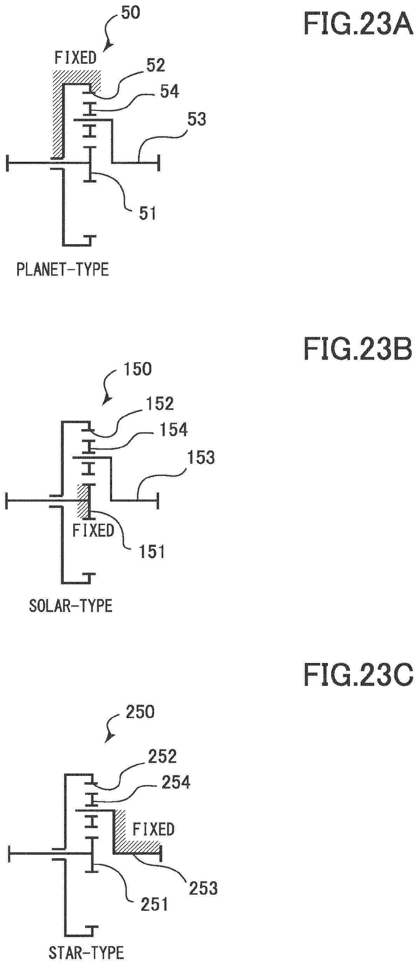

Configurations for decelerating the rotation of the operation portion 400 using other planetary gear mechanisms as modified examples of the first embodiment will be described with reference to FIGs. 23A to 23C. FIG. 23A is a skeleton drawing illustrating the planet-type planetary gear mechanism 50 described with reference to FIGs. 8 to 13. FIG. 23B is a skeleton drawing illustrating a solar-type planetary gear mechanism 150. FIG. 23C is a skeleton drawing illustrating a star-type planetary gear mechanism 250.

The solar-type planetary gear mechanism 150 serving as a modified example of the first embodiment includes, as illustrated in FIG. 23B, a sun gear 151, an internal gear 152, and a planetary carrier 153 that supports a plurality of planetary gears 154 rotatably. In the solar-type planetary gear mechanism 150, the rotation of the operation portion 400 (refer to FIG. 2) is entered to the internal gear 152. Further, the sun gear 151 is fixed to the base unit 200. The internal gear 152 is meshed with the plurality of planetary gears 154, and the planetary carrier 153 rotates in the same direction as the internal gear 152. That is, the internal gear 152 is an input member (input portion) that rotates in response to input of the rotation of the operation portion 400, the sun gear 151 is a fixed member, and the planetary carrier 153 is an output member (output portion) that outputs the transmitted rotation to the pressing member 21cp. The force applied to the operation portion 400 by the user is received by the internal gear 152, and transmitted from the internal gear 152 through the planetary gears 154 to the planetary carrier 153. The sun gear 151 is fixed to the base unit 200 so that the position thereof within the base unit 200 is not varied. According to the solar-type planetary gear mechanism 150, the rotation angle of the planetary carrier 153 is smaller than the rotation angle of the operation portion 400 and the internal gear 152.

Further, the star-type planetary gear mechanism 250 which is another modified example of the first embodiment includes, as illustrated in FIG. 23C, a sun gear 251, an internal gear 252, and a planetary carrier 253 that supports a plurality of planetary gears 254 rotatably. In the star-type planetary gear mechanism 250, the rotation of the operation portion 400 (refer to FIG. 2) is entered to the sun gear 251. Further, the planetary carrier 253 is fixed to the base unit 200. The sun gear 251 is meshed with a plurality of planetary gears 254, and the internal gear 252 is meshed with a plurality of planetary gears 254 and rotates in the opposite direction as the sun gear 251. That is, the sun gear 251 is an input member (input portion) that rotates in response to input of the rotation of the operation portion 400, the planetary carrier 253 is a fixed member, and the internal gear 252 is an output member (output portion) that outputs the transmitted rotation to the pressing member 21cp. The force applied to the operation portion 400 by the user is received by the sun gear 251, and transmitted from the sun gear 251 through the planetary gears 254 to the internal gear 252. The planetary carrier 253 is fixed to the base unit 200 so that the position thereof within the base unit 200 is not varied. According to the star-type planetary gear mechanism 250, the rotation angle of the internal gear 252 is smaller than the rotation angle of the operation portion 400 and the sun gear 251.

The planet-type planetary gear mechanism 50 and the solar-type planetary gear mechanism 150 output the rotation entered from the operation portion 400 as a rotation in the same direction, and the star-type planetary gear mechanism 250 outputs the rotation entered from the operation portion 400 as a rotation in the opposite direction. In any case, the planetary gear mechanisms 50, 150, and 250 serving as the deceleration mechanism may reduce the operation force of the operation portion 400 so as to decelerate the rotation of the operation portion 400 and transmit the same to the gear portion 21cg of the pressing member 21cp. If the numbers of teeth of the respective gears constituting the planet-type, the solar-type, and the star-type planetary gear mechanisms are the same, the planet-type planetary gear mechanism is preferable, since the greatest deceleration ratio may be achieved.

### [Second Embodiment]

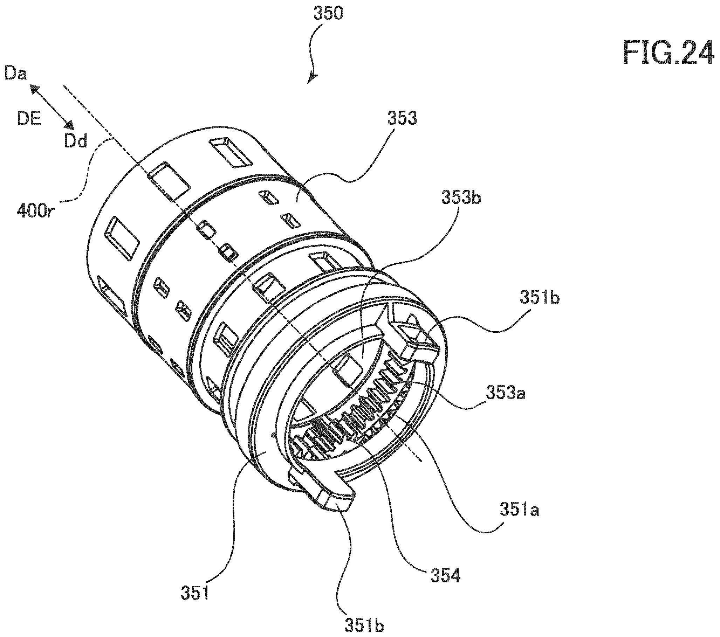

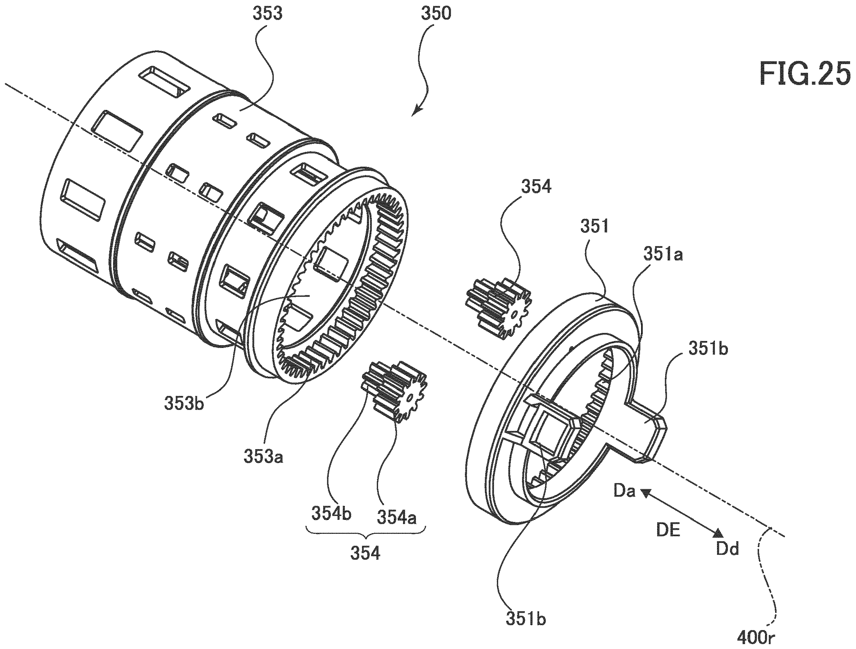

Next, a medical apparatus according to a second embodiment will be described with reference to FIGs. 24 and 25. The present embodiment differs from the first embodiment in that a stepped gear mechanism 350 is adopted instead of the planetary gear mechanism 50. Below, the elements assigned with the same reference numbers as the first embodiment have substantially the same configurations and functions as those described in the first embodiment, and the parts that differ from the first embodiment will mainly be described.

FIG. 24 is a perspective view illustrating the stepped gear mechanism 350. FIG. 25 is an exploded perspective view illustrating the stepped gear mechanism 350. The base unit 200 (refer to FIG. 8) includes the stepped gear mechanism 350 arranged in a vicinity of the base frame 25, as illustrated in FIGs. 24 and 25. The stepped gear mechanism 350 includes an input gear 351, a plurality of (two according to the present embodiment) stepped gears 354 and 354, and an output gear 353 serving as an output member (output portion). The input gear 351 and the output gear 353 are configured rotatably about the rotation shaft 400r of the operation portion 400 in a state where the catheter unit 100 is attached to the base unit 200. The input gear 351 and the output gear 353 are supported rotatably by the base frame 25 and the base casing 200f, for example, but the present technique is not limited thereto, and they may be supported by other members of the base unit 200. Further, the stepped gears 354 and 354 are supported rotatably by the base frame 25.

The input gear 351 includes a gear portion 351a, and one pair of projected portions 351b and 351b that is engageable to one pair of engagement portions 400j (refer to FIG. 10) disposed on the operation portion 400. The projected portions 351b and 351b are extended along the attaching and detaching direction DE that is parallel to the axial direction of the rotation shaft 400r. The output gear 353 includes a gear portion 353a formed on an inner circumference surface 353b and the plurality of teeth portions 29g (refer to FIG. 15). The plurality of teeth portions 29g are arranged downstream of the input gear 351 and the gear portion 353a in the attachment direction Da within the attaching and detaching direction DE.

Each stepped gear 354 includes a first gear portion 354a that meshes with the gear portion 351a of the input gear 351 and a second gear portion 354b that meshes with the gear portion 353a of the output gear 353, and the first gear portion 354a and the second gear portion 354b rotate integrally. The first gear portion 354a has a first number of teeth, and the second gear portion 354b has a second number of teeth that is smaller than the first number of teeth.

When the catheter unit 100 is attached to the base unit 200, projected portions 351b of the input gear 351 are engaged with the engagement portions 400j of the operation portion 400. Thereby, in a case where the operation portion 400 rotates, the rotation of the operation portion 400 is transmitted to the input gear 351 serving as an input member (input portion). The gear portion 351a serving as an input gear portion of the input gear 351 is meshed with the first gear portion 354a of the stepped gear 354, such that the rotation of the input gear 351 is transmitted to the stepped gear 354. Further, the second gear portion 354b of the stepped gear 354 is meshed with the gear portion 353a serving as an output gear portion of the output gear 353, such that the rotation of the stepped gear 354 is transmitted to the output gear 353. The force applied by the user on the operation portion 400 is received by the input gear 351 and transmitted from the input gear 351 through the stepped gear 354 to the output gear 353. According to the present embodiment, the rotation angle of the output gear 353 is smaller than the rotation angle of the operation portion 400 and the input gear 351.

In this state, the number of teeth (second number of teeth) of the second gear portion 354b of the stepped gear 354 is smaller than the number of teeth (first number of teeth) of the first gear portion 354a, such that the rotation of the input gear 351 is decelerated by the stepped gear 354 and transmitted to the output gear 353. The teeth portion 29g provided on the inner circumference surface 353b of the output gear 353 is meshed with the gear portion 21cg of the pressing member 21cp. By the gear portion 21cg being rotated by the teeth portion 29g, the cam 21cc of the pressing member 21cp presses the leaf spring 21ch, and the leaf spring 21ch (the coupling portion 21c) is switched between the fixing state (retaining state) and the releasing state.

As described, according to the present embodiment, the rotation of the operation portion 400 is decelerated by the stepped gear mechanism 350 and transmitted to the gear portion 21cg of the pressing member 21cp such that the state of the leaf spring 21ch may be switched between the fixing state and the releasing state. That is, the stepped gear mechanism 350 is a deceleration mechanism that decelerates and transmits the rotation of the operation portion 400. Therefore, when operating the operation portion 400 from the removal position to the fixed position, the operation portion 400 must be rotated against the urging force of the leaf spring 21ch, but the operation force by the user operating the operation portion 400 may be reduced, and the operability may be improved. Moreover, the stepped gear mechanism 350 may be configured with a small number of components, such that cutting of costs, downsizing, and increasing of durability may be achieved.

It is assumed that the numbers of teeth of the first gear portion 354a and the second gear portion 354b of the stepped gear 354 are respectively z3 and z4, the number of teeth (third number of teeth) of the gear portion 351a of the input gear 351 is z5, and the number of teeth (fourth number of teeth) of the gear portion 353a of the output gear 353 is z6. In this case, the deceleration ratio of the stepped gear mechanism 350 may be calculated by (z3/z5) × (z6/z4). By adjusting the deceleration ratio of the stepped gear mechanism 350, the rotational amount of the operation portion 400 and the operation force of the operation portion 400 by the user may be set arbitrarily. For example, in the present embodiment, z3= 12, z4 = 6, z5 = 56, and z6 = 50, and the deceleration ratio of the stepped gear mechanism 350 is 1.79.

In order to increase the deceleration ratio of the stepped gear mechanism 350, it is preferable to select a gear whose number of teeth of the first gear portion 354a and the second gear portion 354b of the stepped gear 354 is small and the deceleration ratio (z3/z4) of the stepped gear 354 is great. In the present embodiment, the deceleration ratio (z3/z4) of the stepped gear 354 is 2, such that if the gear ratio (z6/z5) of the gear portion 351a of the input gear 351 and the gear portion 353a of the output gear 353 is 0.5 or greater, the stepped gear mechanism 350 may decelerate the rotation of the operation portion 400. That is, the ratio (z6/z5) of the number of teeth of the gear portion 353a with respect to the number of teeth of the gear portion 351a should be greater than a ratio of a ratio (z4/z3) of the number of teeth of the second gear portion 354b with respect to the number of teeth of the first gear portion 354a.

### (Other Embodiments)

In any of the above-described embodiments, the operation portion 400 is configured rotatably about the rotation shaft 400r, but the present technique is not limited thereto. For example, the operation portion 400 may be configured linearly movable in parallel with the rotation shaft 400r (the attaching and detaching direction DE) to enable the coupling portion 21c to be transited between the fixing state and the releasing state. In a configuration where the operation portion 400 moves linearly, the pressing member 21cp that is interlocked with the linear movement (movement) of the operation portion 400 is also preferably moved linearly, and the deceleration mechanism disposed between the operation portion 400 and the pressing member 21cp is also preferably a linear-motion type. Further, in transmitting the movement such as the rotation or linear movement of the operation portion 400 through the deceleration mechanism to the gear portion 21cg, a motion conversion mechanism that converts rotation to and from linear motion may be disposed as required. A motion conversion mechanism for converting rotation into linear motion may be a ball screw, a rack and pinion, a timing belt, etc. A motion conversion mechanism for converting linear motion into rotation may be a slider crank, a rack and pinion, etc.

In any case, the deceleration mechanism such as the above-described planetary gear mechanisms 50, 150, and 250 and the stepped gear mechanism 350 decelerate the movement (including rotation and linear motion) entered from the operation portion 400 and transmits the same to the pressing member 21cp. The deceleration mechanism includes an output member configured to be driven by force transmitted from the operation portion 400 and move the pressing member 21cpp. The output member may be described as a transmission member that transmits the force transmitted from the operation portion 400 toward the pressing member 21cp. In a case where the operation portion 400 moves for a first movement amount, the output member moves for a second movement amount that is smaller than the first movement amount to move the pressing member 21cp. The deceleration mechanism decelerates the movement of the operation portion 400 and transmits the same to the pressing member 21cp so as to switch the coupling portion 21c between the retaining state and the releasing state. More specifically, in a configuration where the operation portion 400 and the output member rotate, if the operation portion 400 rotates for a first rotation angle serving as the first movement amount, the output member rotates for a second rotation angle serving as a second movement amount smaller than the first rotation angle. In a configuration where the operation portion 400 and the output member move linearly, if the operation portion 400 moves for a first movement amount in the linear motion direction, the output member moves for a second movement amount smaller than the first movement amount. In the case of above-described relationship of movement amounts, the deceleration mechanism may be described as having decelerated the movement of the operation portion 400 and transmitted the same to the output member. By the operation of the deceleration mechanism, the moving velocity of the output member becomes small compared to the moving velocity of the operation portion 400, and in contrast, the force that the output member outputs becomes great compared to the force applied to the operation portion 400 from the user. For example, the torque that the output member outputs becomes great compared to the torque for moving the operation portion 400. In any of the above-described embodiments, in a case where the operation portion 400 moves for a first movement amount, the input member moves for a first movement amount. For example, if the operation portion 400 rotates for a first rotation angle serving as the first movement amount, the input member moves for a first rotation angle.

In any of the above-described embodiments, the operation portion 400 was disposed on the catheter unit 100, but the present technique is not limited thereto. For example, the operation portion 400 may be disposed on the base unit 200.

Further, in any of the above-described embodiments, the operation portion 400 and the input member (input portion) were configured as separate members, but the present technique is not limited thereto. For example, the operation portion 400 may be disposed on the base unit 200, and the input member (input portion) may be formed integrally with the operation portion 400.

Further, as described above, the pressing member 21cp is moved by the output member. In any of the above-described embodiments, the pressing member 21cp was moved directly by the output member, but the present technique is not limited thereto. That is, the transmission member may be disposed between the output member and the pressing member 21cp, and the output member may be configured to move the pressing member 21cp through the transmission member.

Further, examples of the deceleration mechanism for decelerating the rotation entered from the operation portion 400 and transmitting the same to the pressing member 21cp are the planetary gear mechanisms 50, 150, and 250 according to the first embodiment and the stepped gear mechanism 350 according to the second embodiment, but the present technique is not limited thereto. For example, a parallel shaft mechanical reduction gear in which spur gears are assembled, a helical reduction gear in which helical gears are assembled, a bevel gear reduction gear in which bevel gears are assembled, and a worm reduction gear in which a worm gear and a worm wheel are assembled, etc. may be used as the deceleration mechanism. Further, the configuration is not limited to the assembly of gears, and a belt pulley may also be adopted as the deceleration mechanism. Further, a linear motion type reduction gear using a fluid and a piston etc. without using a rotary member may be used as the deceleration mechanism.

Further, according to the first embodiment, the rotation of the operation portion 400 was decelerated by the planetary gear mechanism 50 including eight planetary gears 54, but the present technique is not limited thereto. For example, the number of planetary gears 54 may be one to seven, or nine or more. The technique is not limited to a single pinion type planetary gear mechanism, and a double pinion type planetary gear mechanism may also be applied. Further, the respective gears of the planetary gear mechanism 50 may be substituted with rollers, and a planetary roller mechanism may be adopted in which rotation of the respective rollers is transmitted by frictional force.

Further, according to the second embodiment, the rotation of the operation portion 400 was decelerated by the stepped gear mechanism 350 including one pair of stepped gears 354, but the present technique is not limited thereto. In the stepped gear mechanism 350, the rotation of the operation portion 400 may be decelerated by assembling a plurality of stepped gears in series.

According to the respective embodiments described above, examples were illustrated in which a bendable catheter 11 is used as a target object being operated. However, the present technique is not limited thereto, and an articulated robot may be included in the target object being operated. The articulated robot may include, for example, a medical robot arm equipped with a surgical instrument (such as a forceps or a sharp-pointed knife) disposed on a tip thereof.

Moreover, the disclosure of the present embodiments includes exemplary configurations and method examples as follows.
(Configuration 1)
   a driving source;
   a base unit including a coupling portion connected to the driving source;
   a bendable unit removably attached to the base unit, the bendable unit including:
      a bending portion which is bendable; and
      a linear member configured to be coupled to the coupling portion and configured to bend the bending portion by being driven by the driving source through the coupling portion;
   an operation portion; and
   a deceleration mechanism including an output member that is driven by a force transmitted from the operation portion,
   wherein the coupling portion includes:
      a retaining portion configured to be transited between a retaining state in which the retaining portion retains the linear member in a state where the bendable unit is attached to the base unit and a releasing state in which retaining of the linear member is released; and
      a switching portion configured to be moved by the output member and configured to switch the retaining portion between the retaining state and the releasing state, and
   wherein, in a case where the operation portion is moved for a first movement amount, the output member is configured to be moved for a second movement amount that is smaller than the first movement amount.
(Configuration 2) 2. The medical apparatus according to configuration 1,
   wherein the operation portion is configured rotatably, and
   wherein, in a case where the operation portion is rotated for a first rotation angle serving as the first movement amount, the output member is configured to be rotated for a second rotation angle serving as the second movement amount that is smaller than the first rotation angle.
(Configuration 3) 3. The medical apparatus according to configuration 2,
   wherein the deceleration mechanism is a planetary gear mechanism which includes:
      an input portion configured to rotate in response to input of rotation of the operation portion;
      the output member;
      a fixed member fixed to the base unit; and
      a plurality of planetary gears, and
   wherein the output member is configured to output a rotation, transmitted from the input portion, to the switching portion.
(Configuration 4) 4. The medical apparatus according to configuration 3,
   wherein the input portion is a sun gear,
   wherein the output member is a planetary carrier configured to rotatably and revolvably support the plurality of planetary gears that are meshed with the sun gear, and
   wherein the fixed member is an internal gear that is meshed with the plurality of planetary gears.
(Configuration 5) 5. The medical apparatus according to configuration 3,
wherein the input portion is an internal gear,
wherein the output member is a planetary carrier configured to rotatably and revolvably support the plurality of planetary gears that are meshed with the internal gear, and
wherein the fixed member is a sun gear that is meshed with the plurality of planetary gears.
(Configuration 6) 6. The medical apparatus according to configuration 3,
   wherein the input portion is a sun gear,
   wherein the output member is an internal gear that is meshed with the plurality of planetary gears, and
   wherein the fixed member is a planetary carrier configured to rotatably and revolvably support the plurality of planetary gears.
(Configuration 7) 7. The medical apparatus according to configuration 2,
   wherein the deceleration mechanism includes:
      an input portion that is rotated in response to input of a rotation of the operation portion;
      the output member; and
      a stepped gear that is meshed respectively with the input portion and the output member,
   wherein the output member is configured to output a rotation, transmitted from the input portion, to the switching portion, and
   wherein the stepped gear includes:
      a first gear portion that has a first number of teeth and that is meshed with the input portion; and
      a second gear portion that has a second number of teeth smaller than the first number of teeth and that is meshed with the output member.
(Configuration 8) 8. The medical apparatus according to configuration 7,
   wherein the input portion includes an input gear portion that has a third number of teeth and that is meshed with the first gear portion,
   wherein the output member includes an output gear portion that has a fourth number of teeth and that is meshed with the second gear portion, and
   wherein a ratio of the fourth number of teeth to the third number of teeth is greater than a ratio of the second number of teeth to the first number of teeth.
(Configuration 9) 9. The medical apparatus according to any one of configurations 3 to 7, wherein the input portion is configured to be engaged with the operation portion and rotate concentrically and integrally with the operation portion.
(Configuration 10) 10. The medical apparatus according to any one of configurations 3 to 9,
   wherein the switching portion includes:
      a cam portion configured to switch the retaining portion between the retaining state and the releasing state by rotating; and
      a cam gear configured to rotate integrally with the cam portion, and
   wherein the output member includes an output gear that is meshed with the cam gear.
(Configuration 11) 11. The medical apparatus according to any one of configurations 1 to 10,
   wherein the operation portion is disposed on the bendable unit, and
   wherein the deceleration mechanism is disposed on the base unit and is configured to be moved by the operation portion in a state where the bendable unit is attached to the base unit.
(Configuration 12) 12. The medical apparatus according to any one of configurations 1 to 11, wherein the retaining portion is a leaf spring configured to nip and retain the linear member in the retaining state.

### [Industrial Applicability]

The present invention is applicable to a medical apparatus including a catheter unit, for example. Specifically, the present invention is applicable to a medical apparatus in which a catheter unit is configured attachably/detachably with respect to the base unit.

The present invention is not limited to the embodiments described above, and various changes and modifications are enabled without departing from the spirit and scope of the present invention. Therefore, the following claims are attached to clarify the scope of the present invention.

### [Reference Signs List]

1: Medical apparatus/ 12: bending portion! 21c: coupling portion! 21cc: cam portion (cam)/ 21cg: cam gear (gear portion)/ 21ch: retaining portion (leaf spring)/ 21cp: switching portion (pressing member)/ 29g: output gear (teeth portion)/ 50, 150, 250: deceleration mechanism (planetary gear mechanism)/ 51: input portion (sun gear)/ 52: fixed member (internal gear)/ 53, 153: output member (planetary carrier)/ 54: planetary gear/ 152: input portion (internal gear)/ 151: fixed member (sun gear)/ 100: bendable unit (catheter unit)/ 200: base unit/ 251: input portion (sun gear)/ 252: output member (internal gear)/ 253: fixed member (planetary carrier)/ 350: deceleration mechanism (stepped gear mechanism)/ 351: input portion (input gear)/ 351a: input gear portion (gear portion)/ 353a: output gear portion (gear portion)/ 354: stepped gear/ 354a: first gear portion! 354b: second gear portion/ 400: operation portion! M: driving source/ W: linear member (driving wire)

## Claims

1. A medical apparatus comprising:
a driving source;
a base unit including a coupling portion connected to the driving source;
a bendable unit removably attached to the base unit, the bendable unit including:
a bending portion which is bendable; and
a linear member configured to be coupled to the coupling portion and configured to bend the bending portion by being driven by the driving source through the coupling portion;
an operation portion; and
a deceleration mechanism including an output member that is driven by a force transmitted from the operation portion,
wherein the coupling portion includes:
a retaining portion configured to be transited between a retaining state in which the retaining portion retains the linear member in a state where the bendable unit is attached to the base unit and a releasing state in which retaining of the linear member is released; and
a switching portion configured to be moved by the output member and configured to switch the retaining portion between the retaining state and the releasing state, and
wherein, in a case where the operation portion is moved for a first movement amount, the output member is configured to be moved for a second movement amount that is smaller than the first movement amount.

2. The medical apparatus according to claim 1,
wherein the operation portion is configured rotatably, and
wherein, in a case where the operation portion is rotated for a first rotation angle serving as the first movement amount, the output member is configured to be rotated for a second rotation angle serving as the second movement amount that is smaller than the first rotation angle.

3. The medical apparatus according to claim 2,
wherein the deceleration mechanism is a planetary gear mechanism which includes:
an input portion configured to rotate in response to input of rotation of the operation portion;
the output member;
a fixed member fixed to the base unit; and
a plurality of planetary gears, and
wherein the output member is configured to output a rotation, transmitted from the input portion, to the switching portion.

4. The medical apparatus according to claim 3,
wherein the input portion is a sun gear,
wherein the output member is a planetary carrier configured to rotatably and revolvably support the plurality of planetary gears that are meshed with the sun gear, and
wherein the fixed member is an internal gear that is meshed with the plurality of planetary gears.

5. The medical apparatus according to claim 3,
wherein the input portion is an internal gear,
wherein the output member is a planetary carrier configured to rotatably and revolvably support the plurality of planetary gears that are meshed with the internal gear, and
wherein the fixed member is a sun gear that is meshed with the plurality of planetary gears.

6. The medical apparatus according to claim 3,
wherein the input portion is a sun gear,
wherein the output member is an internal gear that is meshed with the plurality of planetary gears, and
wherein the fixed member is a planetary carrier configured to rotatably and revolvably support the plurality of planetary gears.

7. The medical apparatus according to claim 2,
wherein the deceleration mechanism includes:
an input portion that is rotated in response to input of a rotation of the operation portion;
the output member; and
a stepped gear that is meshed respectively with the input portion and the output member,
wherein the output member is configured to output a rotation, transmitted from the input portion, to the switching portion, and
wherein the stepped gear includes:
a first gear portion that has a first number of teeth and that is meshed with the input portion; and
a second gear portion that has a second number of teeth smaller than the first number of teeth and that is meshed with the output member.

8. The medical apparatus according to claim 7,
wherein the input portion includes an input gear portion that has a third number of teeth and that is meshed with the first gear portion,
wherein the output member includes an output gear portion that has a fourth number of teeth and that is meshed with the second gear portion, and
wherein a ratio of the fourth number of teeth to the third number of teeth is greater than a ratio of the second number of teeth to the first number of teeth.

9. The medical apparatus according to claim 3,
wherein the input portion is configured to be engaged with the operation portion and rotate concentrically and integrally with the operation portion.

10. The medical apparatus according to claim 3,
wherein the switching portion includes:
a cam portion configured to switch the retaining portion between the retaining state and the releasing state by rotating; and
a cam gear configured to rotate integrally with the cam portion, and
wherein the output member includes an output gear that is meshed with the cam gear.

11. The medical apparatus according to any one of claims 1 to 10,
wherein the operation portion is disposed on the bendable unit, and
wherein the deceleration mechanism is disposed on the base unit and is configured to be moved by the operation portion in a state where the bendable unit is attached to the base unit.

12. The medical apparatus according to claim 1,
wherein the retaining portion is a leaf spring configured to nip and retain the linear member in the retaining state.
